# EUROPEAN PATENT APPLICATION

(11) **EP 0 779 363 A2**
(43) Date of publication of application: **18.06.1997**
(21) Application number: 96309004.8
(22) Date of filing: 11.12.1996
(51) Int. Cl.: C12N 15/82, C12N 15/54, C12N 9/10, C08B 30/00, C12N 5/10, A01H 5/00

(54) **Improvements in or relating to soluble starch synthase**

(30) Priority: 12.12.1995 GB 9525353
(71) Applicant: National Starch and Chemical Investment Holding Corporation, Wilmington, Delaware 19809 (US)
(72) Inventor: Smith, Alison Mary, Norwich NR2 2BB (GB); Marshall, Jacqueline, Warwick CV34 5SA (GB); Edwards, Elizabeth Ann, Norwich NR9 3DB (GB); Martin, Catherine Rosemary, Norwich NR15 1JW (GB)
(74) Representative: Lipscombe, Martin John

(57) **Abstract**

Disclosed is altered starch extracted from a transformed potato plant or the progeny thereof, which starch, as extracted, has a viscosity onset temperature as determined by differential scanning calorimetry which is reduced by at least 5°C compared to starch extracted from equivalent, non-transformed plants, together with a nucleic acid sequence comprising at least 200bp and exhibiting at least 80% sequence identity with the corresponding region of the DNA sequence shown in Figure 6, operably linked in the sense or antisense orientation to a promoter operable in a plant, and host cells and plants comprising the sequence.

SEQUENCE LISTING

(1) GENERAL INFORMATION:

(i) APPLICANT:
(A) NAME: National Starch and Chemical Investment
Holding Corporation
(B) STREET: Suite 27, 501 Silverside Road
(C) CITY: Wilmington
(D) STATE: Delaware
(E) COUNTRY: U.S.A.
(F) POSTAL CODE (ZIP): DE 19809

(ii) TITLE OF INVENTION: Improvements in or Relating to Soluble
Starch Synthase

(iii) NUMBER OF SEQUENCES: 4

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(2) INFORMATION FOR SEQ ID NO: 1:

(i) SEQUENCE CHARACTERISTICS:
(A) LENGTH: 15 amino acids
(B) TYPE: amino acid
(C) STRANDEDNESS:
(D) TOPOLOGY: linear

(ii) MOLECULE TYPE: peptide

(iii) HYPOTHETICAL: NO

(v) FRAGMENT TYPE: internal

(xi) SEQUENCE DESCRIPTION: SEQ ID NO: 1:

Phe Ile Pro Ile Pro Tyr Thr Ser Glu Asn Val Val Glu Gly Lys
1               5                   10                  15

(2) INFORMATION FOR SEQ ID NO: 2:

(i) SEQUENCE CHARACTERISTICS:
(A) LENGTH: 7 amino acids
(B) TYPE: amino acid
(C) STRANDEDNESS:
(D) TOPOLOGY: linear

(ii) MOLECULE TYPE: peptide

(iii) HYPOTHETICAL: NO

(v) FRAGMENT TYPE: internal

(xi) SEQUENCE DESCRIPTION: SEQ ID NO: 2:

His Ile Pro Val Phe Gly Gly
1               5

(2) INFORMATION FOR SEQ ID NO: 3:

(i) SEQUENCE CHARACTERISTICS:
(A) LENGTH: 4127 base pairs
(B) TYPE: nucleic acid
(C) STRANDEDNESS: single
(D) TOPOLOGY: linear

(ii) MOLECULE TYPE: cDNA

(vi) ORIGINAL SOURCE:
(A) ORGANISM: Solanum tuberosum
(B) STRAIN: Desiree
(F) TISSUE TYPE: tuber

(vii) IMMEDIATE SOURCE:
(A) LIBRARY: lambda gt11

(ix) FEATURE:
(A) NAME/KEY: CDS
(B) LOCATION:143..3835

(ix) FEATURE:
(A) NAME/KEY: sig_peptide
(B) LOCATION:143..322

(ix) FEATURE:
(A) NAME/KEY: mat_peptide
(B) LOCATION:323..3832

(xi) SEQUENCE DESCRIPTION: SEQ ID NO: 3:

GAATTCGCGG CCGCAGATAG TGTGTTGATG AAGGAGAAGA GAGATATTTC ACATGGGATG   60

TTCTATTTGA TTCTGTGGTG AACAAGAGTT TTACAAAGAA CATTCCTTTT TCTTTTTTCC  120

TTGGTTCTTG TGTGGGTCAG CC ATG GAT GTT CCA TTT CCA CTG CAT AGA TCA  172
                         Met Asp Val Pro Phe Pro Leu His Arg Ser
                         -60                 -55

TTG AGT TGC ACA AGT GTC TCC AAT GCA ATA ACC CAC CTC AAG ATC AAA   220
Leu Ser Cys Thr Ser Val Ser Asn Ala Ile Thr His Leu Lys Ile Lys
-50                 -45                 -40                 -35

CCT ATT CTT GGG TTT GTC TCT CAT GGA ACC ACA AGT CTA TCA GTA CAA 268
Pro Ile Leu Gly Phe Val Ser His Gly Thr Thr Ser Leu Ser Val Gln
-30 -25 -20

TCT TCT TCA TGG AGG AAG GAT GGA ATG GTT ACT GGG GTT TCA TTT TCC 316
Ser Ser Ser Trp Arg Lys Asp Gly Met Val Thr Gly Val Ser Phe Ser
-15 -10 -5

ATT TGT GCA AAT TTC TCG GGA AGA AGA CGG AGA AAA GTT TCA ACT CCT 364
Ile Cys Ala Asn Phe Ser Gly Arg Arg Arg Arg Lys Val Ser Thr Pro
1 5 10

AGG AGT CAA GGC TCT TCA CCT AAG GGG TTT GTG CCA AGG AAG CCC TCA 412
Arg Ser Gln Gly Ser Ser Pro Lys Gly Phe Val Pro Arg Lys Pro Ser
15 20 25 30

GGG ATG AGC ACG CAA AGA AAG GTT CAG AAG AGC AAT GGT GAT AAA GAA 460
Gly Met Ser Thr Gln Arg Lys Val Gln Lys Ser Asn Gly Asp Lys Glu
        35                  40                  45

AGT AAA AGT ACT TCA ACA TCT AAA GAA TCT GAA ATT TCC AAC CAG AAG 508
Ser Lys Ser Thr Ser Thr Ser Lys Glu Ser Glu Ile Ser Asn Gln Lys
    50                  55                  60

ACG GTT GAA GCA AGA GTT GAA ACT AGT GAC GAT GAC ACT AAA GGA GTG 556
Thr Val Glu Ala Arg Val Glu Thr Ser Asp Asp Asp Thr Lys Gly Val
65 70 75

GTG AGG GAC CAC AAG TTT CTG GAG GAT GAG GAT GAA ATC AAT GGT TCT 604
Val Arg Asp His Lys Phe Leu Glu Asp Glu Asp Glu Ile Asn Gly Ser
80 85 90

ACT AAA TCA ATA AGT ATG TCA CCT GTT CGT GTA TCA TCT CAA TTT GTT 652
Thr Lys Ser Ile Ser Met Ser Pro Val Arg Val Ser Ser Gln Phe Val
95 100 105 110

GAA AGT GAA GAA ACT GGT GGT GAT GAC AAG GAT GCT GTA AAG TTA AAC 700
Glu Ser Glu Glu Thr Gly Gly Asp Asp Lys Asp Ala Val Lys Leu Asn
        115                 120                 125

AAA TCA AAG AGA TCG GAA GAG AGT GGT TTT ATA ATT GAT TCT GTA ATA 748
Lys Ser Lys Arg Ser Glu Glu Ser Gly Phe Ile Ile Asp Ser Val Ile
    130                 135                 140

AGA GAA CAA AGT GGA TCT CAG GGG GAA ACT AAT GCC AGT AGC AAG GGA 796
Arg Glu Gln Ser Gly Ser Gln Gly Glu Thr Asn Ala Ser Ser Lys Gly
145 150 155

AGC CAT GCT GTG GGT ACA AAA CTT TAT GAG ATA TTG CAG GTG GAT GTT 844
Ser His Ala Val Gly Thr Lys Leu Tyr Glu Ile Leu Gln Val Asp Val
160 165 170

GAG CCA CAA CAA TTG AAA GAA AAT AAT GCT GGG AAT GTT GAA TAC AAA 892
Glu Pro Gln Gln Leu Lys Glu Asn Asn Ala Gly Asn Val Glu Tyr Lys
175 180 185 190

GGA CCT GTA GCA AGT AAG CTA TTG GAA ATT ACT AAG GCT AGT GAT GTG 940
Gly Pro Val Ala Ser Lys Leu Leu Glu Ile Thr Lys Ala Ser Asp Val
        195                 200                 205

GAA CAC ACT GAA AGC AAT GAG ATT GAT GAC TTA GAC ACT AAT AGT TTC 988
Glu His Thr Glu Ser Asn Glu Ile Asp Asp Leu Asp Thr Asn Ser Phe
    210                 215                 220

TTT AAA TCA GAT TTA ATT GAA GAG GAT GAG CCA TTA GCT GCA GGA ACA 1036
Phe Lys Ser Asp Leu Ile Glu Glu Asp Glu Pro Leu Ala Ala Gly Thr
225 230 235

GTG GAG ACT GGA GAT TCT TCT CTA AAC TTA AGA TTG GAG ATG GAA GCA 1084
Val Glu Thr Gly Asp Ser Ser Leu Asn Leu Arg Leu Glu Met Glu Ala
240 245 250

AAT CTA CGT AGG CAG GCT ATA GAA AGG CTT GCC GAG GAA AAT TTA TTG 1132
Asn Leu Arg Arg Gln Ala Ile Glu Arg Leu Ala Glu Glu Asn Leu Leu
255 260 265 270

CAA GGG ATC AGA TTA TTT TGT TTT CCA GAG GTT GTA AAA CCT GAT GAA 1180
Gln Gly Ile Arg Leu Phe Cys Phe Pro Glu Val Val Lys Pro Asp Glu
        275                 280                 285

GAT GTC GAG ATA TTT CTT AAC AGA GGT CTT TCC ACT TTG AAG AAT GAG 1228
Asp Val Glu Ile Phe Leu Asn Arg Gly Leu Ser Thr Leu Lys Asn Glu
    290                 295                 300

TCT GAT GTC TTG ATT ATG GGA GCT TTT AAT GAG TGG CGC TAT AGG TCT 1276
Ser Asp Val Leu Ile Met Gly Ala Phe Asn Glu Trp Arg Tyr Arg Ser
305 310 315

TTT ACT ACA AGG CTA ACT GAG ACT CAT CTC AAT GGA GAT TGG TGG TCT 1324
Phe Thr Thr Arg Leu Thr Glu Thr His Leu Asn Gly Asp Trp Trp Ser
320 325 330

TGC AAG ATC CAT GTT CCC AAG GAA GCA TAC AGG GCT GAT TTT GTG TTT 1372
Cys Lys Ile His Val Pro Lys Glu Ala Tyr Arg Ala Asp Phe Val Phe
335 340 345 350

TTT AAT GGA CAA GAT GTC TAT GAC AAC AAT GAT GGA AAT GAC TTC AGT 1420
Phe Asn Gly Gln Asp Val Tyr Asp Asn Asn Asp Gly Asn Asp Phe Ser
        355                 360                 365

ATA ACT GTG AAA GGT GGT ATG CAA ATC ATT GAC TTT GAA AAT TTC TTG 1468
Ile Thr Val Lys Gly Gly Met Gln Ile Ile Asp Phe Glu Asn Phe Leu
    370                 375                 380

CTT GAG GAG AAA TGG AGA GAA CAG GAG AAA CTT GCT AAA GAA CAA GCT 1516
Leu Glu Glu Lys Trp Arg Glu Gln Glu Lys Leu Ala Lys Glu Gln Ala
385 390 395

GAA AGA GAA AGA CTA GCA GAA GAA CAA AGA CGA ATA GAA GCA GAG AAA 1564
Glu Arg Glu Arg Leu Ala Glu Glu Gln Arg Arg Ile Glu Ala Glu Lys
400 405 410

GCT GAA ATT GAA GCT GAC AGA GCA CAA GCA AAG GAA GAG GCT GCA AAG 1612
Ala Glu Ile Glu Ala Asp Arg Ala Gln Ala Lys Glu Glu Ala Ala Lys
415 420 425 430

AAA AAG AAA GTA TTG CGA GAA TTG ATG GTA AAA GCC ACG AAG ACT CGT 1660
Lys Lys Lys Val Leu Arg Glu Leu Met Val Lys Ala Thr Lys Thr Arg
        435                 440                 445

GAT ATC ACC TGG TAC ATA GAG CCA AGT GAA TTT AAA TGC GAG GAC AAG 1708
Asp Ile Thr Trp Tyr Ile Glu Pro Ser Glu Phe Lys Cys Glu Asp Lys
    450                 455                 460

GTC AGG TTA TAC TAT AAC AAA AGT TCA GGT CCT CTC TCC CAT GCT AAG 1756
Val Arg Leu Tyr Tyr Asn Lys Ser Ser Gly Pro Leu Ser His Ala Lys
465 470 475

GAC TTG TGG ATC CAC GGA GGA TAT AAT AAT TGG AAG GAT GGT TTG TCT 1804
Asp Leu Trp Ile His Gly Gly Tyr Asn Asn Trp Lys Asp Gly Leu Ser
480 485 490

ATT GTC AAA AAG CTT GTT AAA TCT GAG AGA ATA GAT GGT GAT TGG TGG 1852
Ile Val Lys Lys Leu Val Lys Ser Glu Arg Ile Asp Gly Asp Trp Trp
495 500 505 510

TAT ACA GAG GTT GTT ATT CCT GAT CAG GCA CTT TTC TTG GAT TGG GTT 1900
Tyr Thr Glu Val Val Ile Pro Asp Gln Ala Leu Phe Leu Asp Trp Val
        515                 520                 525

TTT GCT GAT GGT CCA CCC AAG CAT GCC ATT GCT TAT GAT AAC AAT CAC 1948
Phe Ala Asp Gly Pro Pro Lys His Ala Ile Ala Tyr Asp Asn Asn His
    530                 535                 540

CGC CAA GAC TTC CAT GCC ATT GTC CCC AAC CAC ATT CCG GAG GAA TTA 1996
Arg Gln Asp Phe His Ala Ile Val Pro Asn His Ile Pro Glu Glu Leu
545 550 555

TAT TGG GTT GAG GAA GAA CAT CAG ATC TTT AAG ACA CTT CAG GAG GAG 2044
Tyr Trp Val Glu Glu Glu His Gln Ile Phe Lys Thr Leu Gln Glu Glu
560 565 570

AGA AGG CTT AGA GAA GCG GCT ATG CGT GCT AAG GTT GAA AAA ACA GCA 2092
Arg Arg Leu Arg Glu Ala Ala Met Arg Ala Lys Val Glu Lys Thr Ala
575 580 585 590

CTT CTG AAA ACT GAA ACA AAG GAA AGA ACT ATG AAA TCA TTT TTA CTG 2140
Leu Leu Lys Thr Glu Thr Lys Glu Arg Thr Met Lys Ser Phe Leu Leu
        595                 600                 605

TCT CAG AAG CAT GTA GTA TAT ACT GAA CCT CTT GAT ATC CAA GCT GGA 2188
Ser Gln Lys His Val Val Tyr Thr Glu Pro Leu Asp Ile Gln Ala Gly
    610                 615                 620

AGC AGC GTC ACA GTT TAC TAT AAT CCC GCC AAT ACA GTA CTT AAT GGT 2236
Ser Ser Val Thr Val Tyr Tyr Asn Pro Ala Asn Thr Val Leu Asn Gly
625 630 635

AAA CCT GAA ATT TGG TTC AGA TGT TCA TTT AAT CGC TGG ACT CAC CGC 2284
Lys Pro Glu Ile Trp Phe Arg Cys Ser Phe Asn Arg Trp Thr His Arg
640 645 650

CTG GGT CCA TTG CCA CCT CAG AAA ATG TCG CCT GCT GAA AAT GGC ACC 2332
Leu Gly Pro Leu Pro Pro Gln Lys Met Ser Pro Ala Glu Asn Gly Thr
655 660 665 670

CAT GTC AGA GCA ACT GTG AAG GTT CCA TTG GAT GCA TAT ATG ATG GAT 2380
His Val Arg Ala Thr Val Lys Val Pro Leu Asp Ala Tyr Met Met Asp
        675                 680                 685

TTT GTA TTT TCC GAG AGA GAA GAT GGT GGG ATT TTT GAC AAT AAG AGC 2428
Phe Val Phe Ser Glu Arg Glu Asp Gly Gly Ile Phe Asp Asn Lys Ser
    690                 695                 700

GGA ATG GAC TAT CAC ATA CCT GTG TTT GGA GGA GTC GCT AAA GAA CCT 2476
Gly Met Asp Tyr His Ile Pro Val Phe Gly Gly Val Ala Lys Glu Pro
705 710 715

CCA ATG CAT ATT GTC CAT ATT GCT GTC GAA ATG GCA CCA ATT GCA AAG 2524
Pro Met His Ile Val His Ile Ala Val Glu Met Ala Pro Ile Ala Lys
720 725 730

GTG GGA GGC CTT GGT GAT GTT GTT ACT AGT CTT TCC CGT GCT GTT CAA 2572
Val Gly Gly Leu Gly Asp Val Val Thr Ser Leu Ser Arg Ala Val Gln
735 740 745 750

GAT TTA AAC CAT AAT GTG GAT ATT ATC TTA CCT AAG TAT GAC TGT TTG 2620
Asp Leu Asn His Asn Val Asp Ile Ile Leu Pro Lys Tyr Asp Cys Leu
        755                 760                 765

AAG ATG AAT AAT GTG AAG GAC TTT CGG TTT CAC AAA AAC TAC TTT TGG 2668
Lys Met Asn Asn Val Lys Asp Phe Arg Phe His Lys Asn Tyr Phe Trp
    770                 775                 780

GGT GGG ACT GAA ATA AAA GTA TGG TTT GGA AAG GTG GAA GGT CTC TCG 2716
Gly Gly Thr Glu Ile Lys Val Trp Phe Gly Lys Val Glu Gly Leu Ser
785 790 795

GTC TAT TTT TTG GAG CCT CAA AAC GGG TTA TTT TCG AAA GGG TGC GTC 2764
Val Tyr Phe Leu Glu Pro Gln Asn Gly Leu Phe Ser Lys Gly Cys Val
800 805 810

TAT GGT TGT AGC AAC GAT GGT GAA CGA TTT GGT TTC TTC TGT CAC GCG 2812
Tyr Gly Cys Ser Asn Asp Gly Glu Arg Phe Gly Phe Phe Cys His Ala
815 820 825 830

GCT TTG GAG TTT CTT CTG CAA GGT GGA TTT AGT CCG GAT ATC ATT CAT 2860
Ala Leu Glu Phe Leu Leu Gln Gly Gly Phe Ser Pro Asp Ile Ile His
        835                 840                 845

TGC CAT GAT TGG TCT AGT GCT CCT GTT GCT TGG CTC TTT AAG GAA CAA 2908
Cys His Asp Trp Ser Ser Ala Pro Val Ala Trp Leu Phe Lys Glu Gln
    850                 855                 860

TAT ACA CAC TAT GGT CTA AGC AAA TCT CGT ATA GTC TTC ACG ATA CAT 2956
Tyr Thr His Tyr Gly Leu Ser Lys Ser Arg Ile Val Phe Thr Ile His
865 870 875

AAT CTT GAA TTT GGG GCA GAT CTC ATT GGG AGA GCA ATG ACT AAC GCA 3004
Asn Leu Glu Phe Gly Ala Asp Leu Ile Gly Arg Ala Met Thr Asn Ala
880 885 890

GAC AAA GCT ACA ACA GTT TCA CCA ACT TAC TCA CAG GAG GTG TCT GGA 3052
Asp Lys Ala Thr Thr Val Ser Pro Thr Tyr Ser Gln Glu Val Ser Gly
895 900 905 910

AAC CCT GTA ATT GCG CCT CAC CTT CAC AAG TTC CAT GGT ATA GTG AAT 3100
Asn Pro Val Ile Ala Pro His Leu His Lys Phe His Gly Ile Val Asn
        915                 920                 925

GGG ATT GAC CCA GAT ATT TGG GAT CCT TTA AAC GAT AAG TTC ATT CCG 3148
Gly Ile Asp Pro Asp Ile Trp Asp Pro Leu Asn Asp Lys Phe Ile Pro
    930                 935                 940

ATT CCG TAC ACT TCA GAA AAC GTT GTT GAG GGC AAA ACA GCA GCC AAG 3196
Ile Pro Tyr Thr Ser Glu Asn Val Val Glu Gly Lys Thr Ala Ala Lys
945 950 955

GAA GCT TTG CAG CGA AAA CTT GGA CTG AAA CAG GCT GAC CTT CCT TTG 3244
Glu Ala Leu Gln Arg Lys Leu Gly Leu Lys Gln Ala Asp Leu Pro Leu
960 965 970

GTA GGA ATT ATC ACC CGC TTA ACT CAC CAG AAA GGA ATC CAC CTC ATT 3292
Val Gly Ile Ile Thr Arg Leu Thr His Gln Lys Gly Ile His Leu Ile
975 980 985 990

AAA CAT GCT ATT TGG CGC ACC TTG GAA CGG AAC GGA CAG GTA GTC TTG 3340
Lys His Ala Ile Trp Arg Thr Leu Glu Arg Asn Gly Gln Val Val Leu
        995                 1000                1005

CTT GGT TCT GCT CCT GAT CCT AGG GTA CAA AAC AAT TTT GTT AAT TTG 3388
Leu Gly Ser Ala Pro Asp Pro Arg Val Gln Asn Asn Phe Val Asn Leu
    1010                1015                1020

GCA AAT CAA TTG CAC TCC AAA TAT AAT GAC CGC GCA CGA CTC TGT CTA 3436
Ala Asn Gln Leu His Ser Lys Tyr Asn Asp Arg Ala Arg Leu Cys Leu
1025 1030 1035

ACA TAT GAC GAG CCA CTT TCT CAC CTG ATA TAT GCT GGT GCT GAT TTT 3484
Thr Tyr Asp Glu Pro Leu Ser His Leu Ile Tyr Ala Gly Ala Asp Phe
1040 1045 1050

ATT CTA GTT CCT TCA ATA TTT GAG CCA TGT GGA CTA ACA CAA CTT ACC 3532
Ile Leu Val Pro Ser Ile Phe Glu Pro Cys Gly Leu Thr Gln Leu Thr
1055 1060 1065 1070

GCT ATG AGA TAT GGT TCA ATT CCA GTC GTG CGT AAA ACT GGA GGA CTT 3580
Ala Met Arg Tyr Gly Ser Ile Pro Val Val Arg Lys Thr Gly Gly Leu
        1075                1080                1085

TAT GAT ACT GTA TTT GAT GTT GAC CAT GAC AAA GAG AGA GCA CAA CAG 3628
Tyr Asp Thr Val Phe Asp Val Asp His Asp Lys Glu Arg Ala Gln Gln
    1090                1095                1100

TGT GGT CTT GAA CCA AAT GGA TTC AGC TTT GAT GGA GCA GAT GCT GGC 3676
Cys Gly Leu Glu Pro Asn Gly Phe Ser Phe Asp Gly Ala Asp Ala Gly
1105 1110 1115

GGA GTT GAT TAT GCT CTG AAT AGA GCT CTC TCT GCT TGG TAC GAT GGT 3724
Gly Val Asp Tyr Ala Leu Asn Arg Ala Leu Ser Ala Trp Tyr Asp Gly
1120 1125 1130

CGG GAT TGG TTC AAC TCT TTA TGC AAG CAG GTC ATG GAA CAA GAT TGG 3772
Arg Asp Trp Phe Asn Ser Leu Cys Lys Gln Val Met Glu Gln Asp Trp
1135 1140 1145 1150

TCT TGG AAC CGA CCT GCT CTT GAT TAT TTG GAG CTT TAC CAT GCT GCT 3820
Ser Trp Asn Arg Pro Ala Leu Asp Tyr Leu Glu Leu Tyr His Ala Ala
        1155                1160                1165

AGA AAG TTA GAA TAG TTAGTTTGTG AGATGCTAGC AGAAAAATTC ACGAGATCTG 3875
Arg Lys Leu Glu *
    1170

CAATCTGTAC AGGTTCAGTG TTTGGGTCTG GACAGCTTTA TCATTTCCTA TATCAAAGTA 3935

TAAATCAAGT CTACACTGAG GATCAATAGC AGACAGTCCT CAAGTTCATT TCATTTTTTG 3995

GGGCAAACAT ATGAAAGAGC TTAGCCTCTT AATAATGTCG GCCTATTGAT GATTATTTGT 4055

TTTGGGAAGA AATGAGAAAT CAAAGGATGC AAAATAAAAA AAAAAAAAAA AAAAAAAACT 4115

CGTGCCGAAT TC 4127

(2) INFORMATION FOR SEQ ID NO: 4:

(i) SEQUENCE CHARACTERISTICS:
(A) LENGTH: 1231 amino acids
(B) TYPE: amino acid
(D) TOPOLOGY: linear

(ii) MOLECULE TYPE: protein
(xi) SEQUENCE DESCRIPTION: SEQ ID NO: 4:

Met Asp Val Pro Phe Pro Leu His Arg Ser Leu Ser Cys Thr Ser Val
-60 -55 -50 -45

Ser Asn Ala Ile Thr His Leu Lys Ile Lys Pro Ile Leu Gly Phe Val
-40 -35 -30

Ser His Gly Thr Thr Ser Leu Ser Val Gln Ser Ser Ser Trp Arg Lys
-25 -20 -15

Asp Gly Met Val Thr Gly Val Ser Phe Ser Ile Cys Ala Asn Phe Ser
-10 -5 1

Gly Arg Arg Arg Arg Lys Val Ser Thr Pro Arg Ser Gln Gly Ser Ser
5                   10                  15                  20

Pro Lys Gly Phe Val Pro Arg Lys Pro Ser Gly Met Ser Thr Gln Arg
                25                  30                  35

Lys Val Gln Lys Ser Asn Gly Asp Lys Glu Ser Lys Ser Thr Ser Thr
            40                  45                  50

Ser Lys Glu Ser Glu Ile Ser Asn Gln Lys Thr Val Glu Ala Arg Val
        55                  60                  65

Glu Thr Ser Asp Asp Asp Thr Lys Gly Val Val Arg Asp His Lys Phe
    70                  75                  80

Leu Glu Asp Glu Asp Glu Ile Asn Gly Ser Thr Lys Ser Ile Ser Met
85                  90                  95                  100

Ser Pro Val Arg Val Ser Ser Gln Phe Val Glu Ser Glu Glu Thr Gly
                105                 110                 115

Gly Asp Asp Lys Asp Ala Val Lys Leu Asn Lys Ser Lys Arg Ser Glu
            120                 125                 130

Glu Ser Gly Phe Ile Ile Asp Ser Val Ile Arg Glu Gln Ser Gly Ser
        135                 140                 145

Gln Gly Glu Thr Asn Ala Ser Ser Lys Gly Ser His Ala Val Gly Thr
    150                 155                 160

Lys Leu Tyr Glu Ile Leu Gln Val Asp Val Glu Pro Gln Gln Leu Lys
165                 170                 175                 180

Glu Asn Asn Ala Gly Asn Val Glu Tyr Lys Gly Pro Val Ala Ser Lys
                185                 190                 195

Leu Leu Glu Ile Thr Lys Ala Ser Asp Val Glu His Thr Glu Ser Asn
            200                 205                 210

Glu Ile Asp Asp Leu Asp Thr Asn Ser Phe Phe Lys Ser Asp Leu Ile
        215                 220                 225

Glu Glu Asp Glu Pro Leu Ala Ala Gly Thr Val Glu Thr Gly Asp Ser
    230                 235                 240

Ser Leu Asn Leu Arg Leu Glu Met Glu Ala Asn Leu Arg Arg Gln Ala
245                 250                 255                 260

Ile Glu Arg Leu Ala Glu Glu Asn Leu Leu Gln Gly Ile Arg Leu Phe
                265                 270                 275

Cys Phe Pro Glu Val Val Lys Pro Asp Glu Asp Val Glu Ile Phe Leu
            280                 285                 290

Asn Arg Gly Leu Ser Thr Leu Lys Asn Glu Ser Asp Val Leu Ile Met
        295                 300                 305

Gly Ala Phe Asn Glu Trp Arg Tyr Arg Ser Phe Thr Thr Arg Leu Thr
    310                 315                 320

Glu Thr His Leu Asn Gly Asp Trp Trp Ser Cys Lys Ile His Val Pro
325                 330                 335                 340

Lys Glu Ala Tyr Arg Ala Asp Phe Val Phe Phe Asn Gly Gln Asp Val
                345                 350                 355

Tyr Asp Asn Asn Asp Gly Asn Asp Phe Ser Ile Thr Val Lys Gly Gly
            360                 365                 370

Met Gln Ile Ile Asp Phe Glu Asn Phe Leu Leu Glu Glu Lys Trp Arg
        375                 380                 385

Glu Gln Glu Lys Leu Ala Lys Glu Gln Ala Glu Arg Glu Arg Leu Ala
    390                 395                 400

Glu Glu Gln Arg Arg Ile Glu Ala Glu Lys Ala Glu Ile Glu Ala Asp
405                 410                 415                 420

Arg Ala Gln Ala Lys Glu Glu Ala Ala Lys Lys Lys Lys Val Leu Arg
                425                 430                 435

Glu Leu Met Val Lys Ala Thr Lys Thr Arg Asp Ile Thr Trp Tyr Ile
            440                 445                 450

Glu Pro Ser Glu Phe Lys Cys Glu Asp Lys Val Arg Leu Tyr Tyr Asn
        455                 460                 465

Lys Ser Ser Gly Pro Leu Ser His Ala Lys Asp Leu Trp Ile His Gly
    470                 475                 480

Gly Tyr Asn Asn Trp Lys Asp Gly Leu Ser Ile Val Lys Lys Leu Val
485                 490                 495                 500

Lys Ser Glu Arg Ile Asp Gly Asp Trp Trp Tyr Thr Glu Val Val Ile
                505                 510                 515

Pro Asp Gln Ala Leu Phe Leu Asp Trp Val Phe Ala Asp Gly Pro Pro
            520                 525                 530

Lys His Ala Ile Ala Tyr Asp Asn Asn His Arg Gln Asp Phe His Ala
        535                 540                 545

Ile Val Pro Asn His Ile Pro Glu Glu Leu Tyr Trp Val Glu Glu Glu
    550                 555                 560

His Gln Ile Phe Lys Thr Leu Gln Glu Glu Arg Arg Leu Arg Glu Ala
565                 570                 575                 580

Ala Met Arg Ala Lys Val Glu Lys Thr Ala Leu Leu Lys Thr Glu Thr
                585                 590                 595

Lys Glu Arg Thr Met Lys Ser Phe Leu Leu Ser Gln Lys His Val Val
            600                 605                 610

Tyr Thr Glu Pro Leu Asp Ile Gln Ala Gly Ser Ser Val Thr Val Tyr
        615                 620                 625

Tyr Asn Pro Ala Asn Thr Val Leu Asn Gly Lys Pro Glu Ile Trp Phe
    630                 635                 640

Arg Cys Ser Phe Asn Arg Trp Thr His Arg Leu Gly Pro Leu Pro Pro
645                 650                 655                 660

Gln Lys Met Ser Pro Ala Glu Asn Gly Thr His Val Arg Ala Thr Val
                665                 670                 675

Lys Val Pro Leu Asp Ala Tyr Met Met Asp Phe Val Phe Ser Glu Arg
            680                 685                 690

Glu Asp Gly Gly Ile Phe Asp Asn Lys Ser Gly Met Asp Tyr His Ile
        695                 700                 705

Pro Val Phe Gly Gly Val Ala Lys Glu Pro Pro Met His Ile Val His
    710                 715                 720

Ile Ala Val Glu Met Ala Pro Ile Ala Lys Val Gly Gly Leu Gly Asp
725                 730                 735                 740

Val Val Thr Ser Leu Ser Arg Ala Val Gln Asp Leu Asn His Asn Val
                745                 750                 755

Asp Ile Ile Leu Pro Lys Tyr Asp Cys Leu Lys Met Asn Asn Val Lys
            760                 765                 770

Asp Phe Arg Phe His Lys Asn Tyr Phe Trp Gly Gly Thr Glu Ile Lys
        775                 780                 785

Val Trp Phe Gly Lys Val Glu Gly Leu Ser Val Tyr Phe Leu Glu Pro
    790                 795                 800

Gln Asn Gly Leu Phe Ser Lys Gly Cys Val Tyr Gly Cys Ser Asn Asp
805                 810                 815                 820

Gly Glu Arg Phe Gly Phe Phe Cys His Ala Ala Leu Glu Phe Leu Leu
                825                 830                 835

Gln Gly Gly Phe Ser Pro Asp Ile Ile His Cys His Asp Trp Ser Ser
            840                 845                 850

Ala Pro Val Ala Trp Leu Phe Lys Glu Gln Tyr Thr His Tyr Gly Leu
        855                 860                 865

Ser Lys Ser Arg Ile Val Phe Thr Ile His Asn Leu Glu Phe Gly Ala
    870                 875                 880

Asp Leu Ile Gly Arg Ala Met Thr Asn Ala Asp Lys Ala Thr Thr Val
885                 890                 895                 900

Ser Pro Thr Tyr Ser Gln Glu Val Ser Gly Asn Pro Val Ile Ala Pro
                905                 910                 915

His Leu His Lys Phe His Gly Ile Val Asn Gly Ile Asp Pro Asp Ile
            920                 925                 930

Trp Asp Pro Leu Asn Asp Lys Phe Ile Pro Ile Pro Tyr Thr Ser Glu
        935                 940                 945

Asn Val Val Glu Gly Lys Thr Ala Ala Lys Glu Ala Leu Gln Arg Lys
    950                 955                 960

Leu Gly Leu Lys Gln Ala Asp Leu Pro Leu Val Gly Ile Ile Thr Arg
965                 970                 975                 980

Leu Thr His Gln Lys Gly Ile His Leu Ile Lys His Ala Ile Trp Arg
                985                 990                 995

Thr Leu Glu Arg Asn Gly Gln Val Val Leu Leu Gly Ser Ala Pro Asp
            1000                1005                1010

Pro Arg Val Gln Asn Asn Phe Val Asn Leu Ala Asn Gln Leu His Ser
        1015                1020                1025

Lys Tyr Asn Asp Arg Ala Arg Leu Cys Leu Thr Tyr Asp Glu Pro Leu
    1030                1035                1040

Ser His Leu Ile Tyr Ala Gly Ala Asp Phe Ile Leu Val Pro Ser Ile
1045                1050                1055                1060

Phe Glu Pro Cys Gly Leu Thr Gln Leu Thr Ala Met Arg Tyr Gly Ser
                1065                1070                1075

Ile Pro Val Val Arg Lys Thr Gly Gly Leu Tyr Asp Thr Val Phe Asp
            1080                1085                1090

Val Asp His Asp Lys Glu Arg Ala Gln Gln Cys Gly Leu Glu Pro Asn
        1095                1100                1105

Gly Phe Ser Phe Asp Gly Ala Asp Ala Gly Gly Val Asp Tyr Ala Leu
    1110                1115                1120

Asn Arg Ala Leu Ser Ala Trp Tyr Asp Gly Arg Asp Trp Phe Asn Ser
1125                1130                1135                1140

Leu Cys Lys Gln Val Met Glu Gln Asp Trp Ser Trp Asn Arg Pro Ala
                1145                1150                1155

Leu Asp Tyr Leu Glu Leu Tyr His Ala Ala Arg Lys Leu Glu  *
            1160                1165                1170

## Description

### Field of the Invention

This invention relates, *inter alia,* to a soluble enzyme, obtainable from potato tubers, having starch synthase activity, to nucleic acid sequences encoding the same, to constructs and transgenic plants comprising the nucleic acid sequences, to a method of altering the starch composition of a plant, and to altered starch obtainable from a transgenic plant.

### Background of the Invention

In the storage organs of most species of plants multiple forms of both granule-bound and soluble starch synthases have been found (for review, see Smith & Martin 1993, In: Biosynthesis and manipulation of plant products (D. Grierson, Ed.) Blackie Academic and Professional (Glasgow), pp1-54). In most cases it is not known whether these forms are distinct gene products and, for the most part, what their detailed functions are. The exception to this is in the case of a widely-distributed and highly conserved class of granule-bound starch synthases of approximately 60 kDa, which are collectively referred to as granule-bound starch synthase I (GBSS I; Martin & Smith, 1995 Plant Cell 7. 971-985). Experiments with the waxy and amf mutants of cereals and potatoes respectively (Macdonald & Preiss, 1985 Plant Physiol. 78, 849-852 1985; Hovenkamp-Hermelink *et al.,* 1987 Theor. Appl. Genet. 7, 217-221) and antisense potato plants (Visser *et al.,* 1991 Mol. Gen. Genet. 22, 289-296, Kuipers *et al.,* 1994 Plant Cell 6, 43-52) have shown that when the level of GBSS I protein is reduced, the ratio of amylose to amylopectin in the starch is also reduced. Where GBSS I is absent, the starch contains only amylopectin. This suggests that GBSS I is responsible for amylose synthesis.

However, the detailed functions of other isoforms of starch synthase are as yet unknown. In general, in conjunction with starch branching enzyme, they must be responsible for amylopectin synthesis but it is unknown whether different isoforms make different contributions to its structure. The first step in trying to understand the functions of these starch synthases is to characterise all of the isoforms in one organ. A few isoforms of starch synthase, other than GBSS I, have been identified at a detailed biochemical and molecular level in pea (Smith, 1990 Planta *182,* 599-604; Denyer & Smith 1992 Planta *186,* 609-617, Dry *et al.* 1992 Planta *186,* 609-617) and rice (Baba *et al.,* 1993 Plant Physiol. *103,* 565-573), and at a detailed biochemical level in maize (Mu *et al.,* 1994 Plant J. 6, 151-159) and wheat (Denyer *et al.,* 1995 Planta 196, 256-265). However only in the case of pea and maize has the quantitative importance of the isoforms been estimated. A complete picture of the role and importance of all the isoforms of starch synthase is not available for any other storage organ.

Carbohydrate metabolism and starch synthesis has been extensively studied in potato tuber (Hajirezaei *et al.,* 1993 Planta *192,* 16-30; Geigenberger & Stitt 1993 Planta *189,* 329-339; Geigenberger *et al.,* 1994 Planta 193, 486-493; Sonnewald *et al.,* 1994 Plant Cell Environ. 17, 649-658) and this organ has great potential as a source of commercially important starches created through genetic manipulation (Shewmaker & Stalker 1992 Plant Physiol. *100,* 1083-1086; Visser & Jacobsen 1993 Trends Biotech. *11*, 63-68; Muller-Rober & Koßmann, 1994 Plant Cell Environ. *17*, 601-613). One of the major gaps in understanding starch synthesis in this organ and hence in the ability to manipulate its starch in useful ways, is the nature of its starch synthases.

In potato, until recently, only two starch synthases have been characterised in any great detail; GBSS I and GBSS II. GBSS I is exclusively granule-bound, it has a molecular weight of 59 kDa. The gene has been cloned and its predicted amino acid sequence is very similar to that of the waxy gene product in cereals (Vos-Scheperkeuter *et al.,* 1986 Plant Physiol. 82, 411 4 16; van der Leij *et al.,* 1991 Mol. Gen. Genet. 228, 240-248). GBSS II has an apparent molecular weight, as judged by SDS-PAGE, of 92 kDa and it is both bound into the starch granule and present as a soluble form. Its predicted amino acid sequence (having an expected molecular weight of 80kDa) is similar to GBSS II in pea embryos, an isoform which accounts for 60-70 % of the soluble starch synthase activity of the pea embryo (Denyer & Smith 1992 cited above). However, GBSS II accounts for only approximately 10-15% of the total soluble starch synthase activity in potato tubers (Edwards *et al.,* 1995 Plant J. 8, 283-294).

There have been several reported characterisations of the starch synthases found in the soluble fraction of potato tubers (Frydman & Cardini 1966 Arch. Biochem. Biophys. *116,* 9-18; Catz *et al.,* 1989 An. Asoc. Quim. Argent. 77, 47-51) and a few attempts have been made to purify the major soluble starch synthases (Hawker *et al.,* 1972. Phytochem. *11,* 1278- 1293; Baba *et al.,* 1990 Phytochem. 29, 719-723; Ponstein 1990. Starch synthesis in potato tubers. Ph. D. Thesis, State University Groningen, The Netherlands). These reports disagree on both the number of soluble starch synthases and their molecular weights. The quantitative contribution of the putative forms is not known, and where multiple forms are postulated, it is not known whether they are independent gene products.

After the priority date of the present application, two publications have been made which provide information about a further starch synthase found in potato. One of these publications is by the present inventors (Marshall *et al.,* 1996 The Plant Cell 8, 1121-1135). The other publication is PCT patent application WO 96/15248 (published 23rd May 1996), in the name of Institut Für Genbiologische Forschung Berlin GMBH. The PCT application includes the European Patent Office in the list of designated territories.

WO 96/15248 discloses the nucleotide sequence of a full length cDNA clone ("SSSA") said to encode an isoform of soluble starch synthase enzyme from potato, together with the predicted amino acid sequence of the enzyme. The application further discloses the use of a 1.2kb portion of the cDNA clone, operably linked in the antisense orientation to the CaMV 35S promoter, to transform potato plants. In addition WO 96/15248 discloses the sequence of a cDNA clone ("SSSB") said to encode a second isoform of the potato soluble starch synthase. Similarly, a portion (1.8kb) of this sequence was introduced into potato plants in the antisense orientation.

In fact, the present inventors have found that the nucleotide sequence disclosed in WO 96/15248 contains an error, causing a frame shift, such that most of the predicted amino acid sequence is incorrect.

It was found that the transformed plants disclosed in WO 96/15248 had reduced enzyme activity. Starch obtained from the tubers of the transformed plants was found to have altered properties compared to starch obtained from control wild type plants. It was stated that the starch from the transformed plants exhibited a lower viscosity onset temperature than starch from control plants. (By way of explanation, when aqueous suspensions of starch granules are heated, the granules swell and absorb water, in a process known as gelatinisation. A number of techniques are available for the analysis of gelatinisation, a particularly convenient method being differential scanning calorimetry or the viscoamylograph, in which the viscosity of a stirred starch suspension is monitored under a defined temperature/time regime. Such analysis typically shows a particular temperature, the "viscosity onset temperature", at which the process of gelatinisation begins and which causes a marked increase in viscosity of the starch suspension).

In a few instances, the transformed plants disclosed in WO 96/15248 gave rise to starch in which the "Verkleisterungstemperatur" (equivalent to the viscosity onset temperature, V) was 2 to 3°C lower compared to starch from equivalent, but untransformed, plants. However, it is apparent that the results of subsequent experiments (described in example 13 in the document) gave a value of V for starch from control plants which was lower than that found for starch from transformed plants in previous experiments. Accordingly, the person skilled in the art is not able to deduce that starch from the transformed plants described in WO 96/15248 displayed a viscosity onset temperature which was consistently significantly lower than that of control plants.

### Summary of the Invention

In a first aspect the invention provides a polypeptide obtainable from a soluble extract of potato tubers and having starch synthase activity, in substantially isolated form.

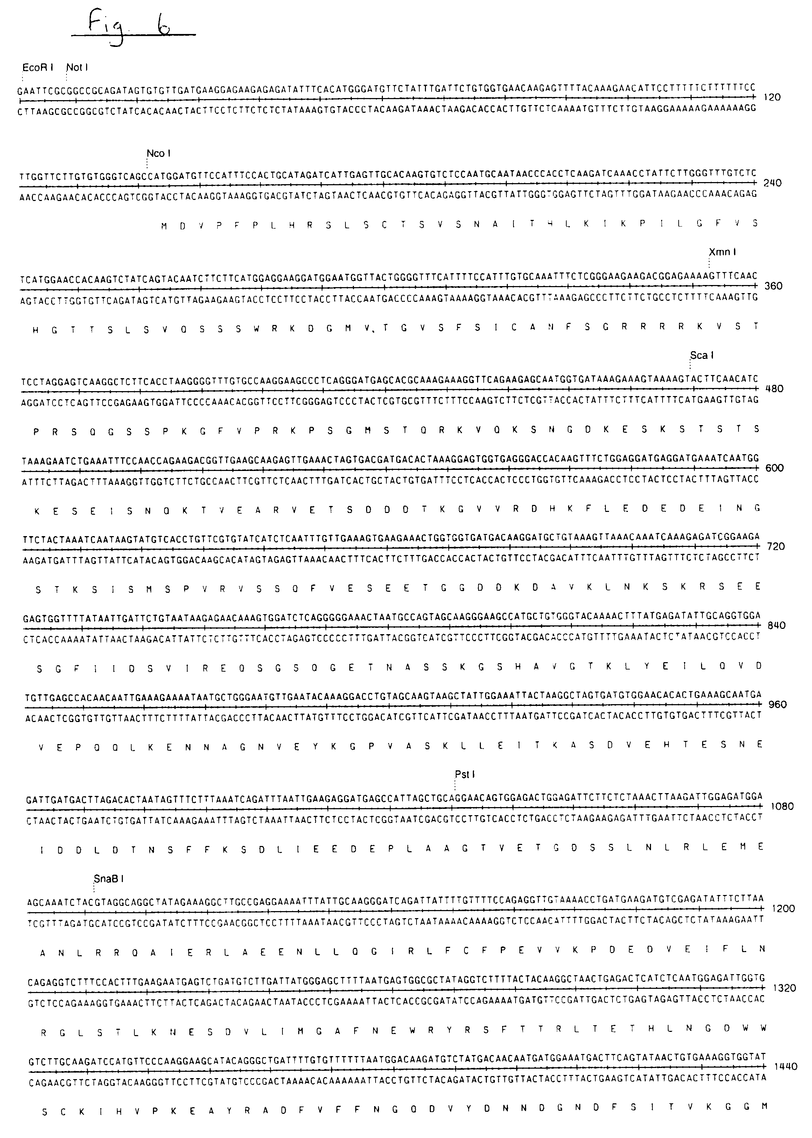

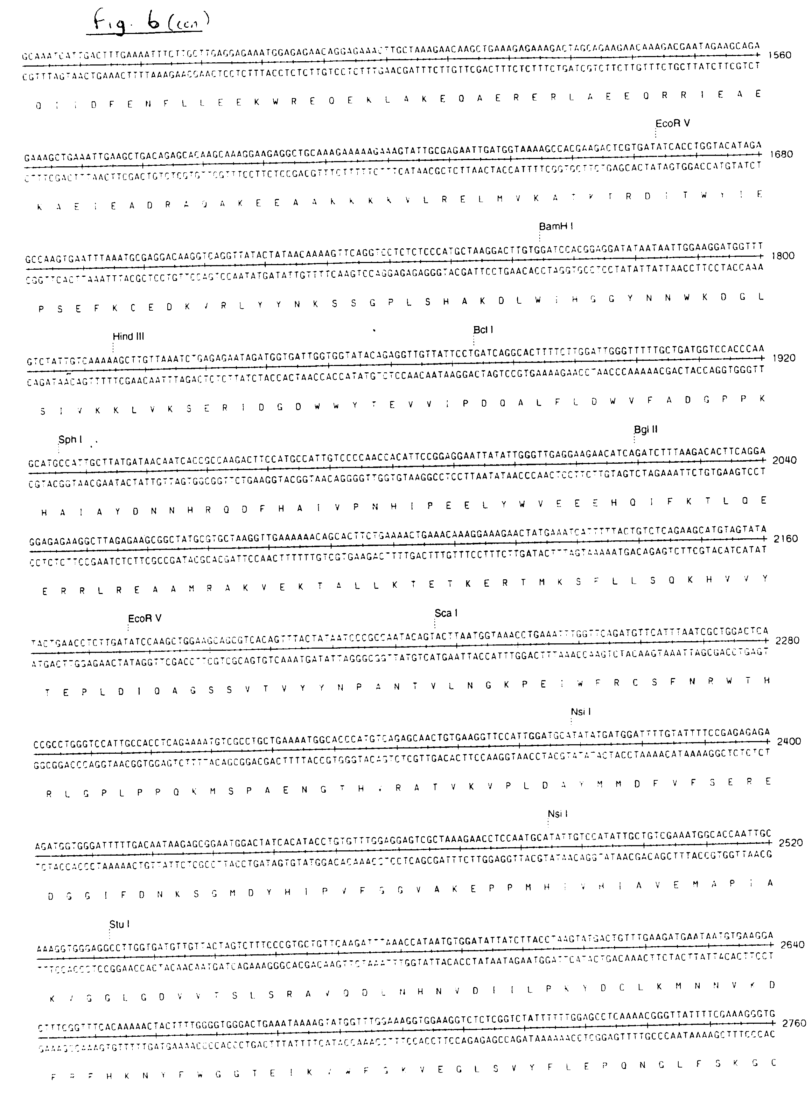

Typically the polypeptide will have an apparent molecular weight, as judged by SDS-PAGE, in the range 100-140 kDa, or will be a functional equivalent of such a polypeptide. More particularly, the polypeptide may have an apparent molecular weight of 140, 120 or 110 kDa. Particular functional equivalents envisaged are breakdown products of the polypeptide, which seem to occur naturally. Another particular functional equivalent is the polypeptide obtainable from developing tubers of the Desiree cultivar, which polypeptide has an apparent molecular weight, as judged by SDS-PAGE, of 100 kDa. Typically the polypeptide will comprise the amino acid sequence shown in Figure 6.

In another aspect the invention provides a nucleic acid sequence directing the expression of at least a portion of one of the polypeptides defined above. Preferably the sequence comprises at least 200-300bp, more preferably at least 300-600bp, and most preferably in excess of 600bp. Typically the nucleic acid sequence will comprise the nucleotide sequence shown in Figure 6, although those skilled in the art will appreciate that, due to the degeneracy of the genetic code, a nucleotide sequence substantially different to that shown in Figure 6 may encode a polypeptide having substantially the same amino acid sequence as that shown in Figure 6. Such nucleic acid sequences are to be considered as functional equivalents and thus fall within the scope of the present invention. Other functional equivalents are those nucleic acid sequences which are not substantially different and which may hybridise, under standard laboratory hybridisation conditions, to either strand of the nucleotide sequence shown in Figure 6.

Comparison with known starch synthase sequences, with the benefit of the disclosure herein, will enable those skilled in the art to identify regions of the sequence shown in Figure 6 which are not evolutionarily conserved, and so more amenable to alteration (e.g. addition, deletion or substitutions), whilst retaining functional equivalence.

Desirably such functional equivalents will possess at least 80% sequence identity, preferably at least 85% sequence identity, and more preferably at least 90% sequence identity with the nucleotide sequence shown in Figure 6. Desirably the nucleotide sequence of the invention, or a functional equivalent sequence will, when introduced into a suitable plant in a suitable manner (known to those skilled in the art), alter the synthesis of starch in the plant.

For the purposes of the present specification, the sequences encoding polypeptides with starch synthase activity, or portions of such sequences, disclosed in WO 96/15248 are not considered as functional equivalents of the sequence shown in Figure 6.

In a particular embodiment, the invention provides a nucleic acid sequence comprising at least 200 bp and exhibiting at least 80% sequence identity with the corresponding region of the DNA sequence shown in Figure 6, operably linked in the sense or antisense orientation to a promoter operable in a plant.

Those skilled in the art will readily be able to conduct a sequence alignment between the other sequence and that detailed in Figure 6. The % identity of the two sequences is to be compared in those regions which are aligned by readily available computer programs (e.g. MegAlign), which align corresponding regions of sequences. Advantageously the % identity between the two sequences will be at least 85%, preferably at least 90%, and the corresponding region of the sequence shown in Figure 6 may comprise a 5' and/or a 3' untranslated region ("UTR") and/or a translated region.

Thus, in another aspect the invention provides a nucleic acid construct (typically DNA) comprising the nucleic acid sequence of the invention in operable linkage to a promoter active in a plant. The nucleic acid sequence may be operably linked to the promoter in either the sense or the anti-sense orientation. Anti-sense methods are well known in altering one or more characteristics of a plant into which the anti-sense sequence is inserted (see for example EP-A-0 458 367, EP-B-0 240 208 and US 5, 107, 065). Similarly, "sense suppression" is a method which is becoming increasingly widely adopted and documented (for a review, see Matzke & Matzke 1995 Plant Physiology *107,* 679-685). Either approach could be used with the nucleic acid sequence of the invention, so as to alter one or more characteristics of a plant into which the sequence was introduced. Those skilled in the art wil be aware that anti-sense inhibition or sense suppression may be achieved by the use of 5' or 3'non-translated portions of the relevant gene, or use of coding portions of the gene, or any combination thereof.

It is believed that antisense methods are mainly operable by the production of antisense mRNA which hybridises to the sense mRNA, preventing its translation into functional polypeptide, possibly by causing the hybrid RNA to be degraded (e.g. Sheehy *et al.,* 1988 PNAS 85, 8805-8809; Van der Krol *et al.,* Mol. Gen. Genet. 220, 204-212). Sense suppression also requires homology between the introduced sequence and the target gene, but the exact mechanism is unclear. It is apparent however that, in relation to both antisense and sense suppression, neither a full length nucleotide sequence, nor a "native" sequence is essential. Preferably the nucleic acid sequence used in the method will comprise at least 200-300bp, more preferably at least 300-600bp, of the full length sequence, but by simple trial and error other fragments (smaller or larger) may be found which are functional in altering the characteristics of the plant. It is also known that untranslated portions of sequence can suffice to inhibit expression of the homologous gene - coding portions may be present within the introduced sequence, but they do not appear to be essential under all circumstances.

The invention further provides a host cell into which has been introduced a nucleic acid sequence in accordance with the invention defined above. Typically the host cell will be a plant cell, and conveniently the sequence is introduced in a nucleic acid construct and subsequently integrated into the host cell genome.

In a further aspect the invention provides a plant or part thereof (e.g. plant cell), into which has been introduced a sequence in accordance with the invention, or the progeny of such a plant or part thereof. Desirably the plant or part thereof into which the sequence is introduced, will comprise a natural gene which shares sequence homology with the introduced sequence. In preferred embodiments the introduced sequence will exhibit at least 70% homology with a starch synthase gene naturally present in the plant or part thereof, although the level of homology may be increased with advantage, such that the expression of the gene product of the naturally present gene in the plant is substantially inhibited. Conveniently the sequence of the invention will be introduced as part of a nucleic acid construct, as described above. Typically the plant will be one of commercial significance, such as one of the following: potato, tomato, rice, wheat, pea, cassava, sweet potato, barley, oat and maize.

Those skilled in the art will appreciate that introduction of the nucleic acid sequence of the invention into a plant might alter the starch composition thereof. In another aspect therefore the invention provides altered starch extracted from a plant into which has been introduced the nucleic acid sequence of the invention, or altered starch extracted from the progeny of such a plant. The invention also provides a method of altering one or more characteristics of a plant, comprising introducing into the plant a nucleic acid sequence in accordance with the invention.

In particular the invention provides altered starch extracted from a transformed potato plant or the progeny thereof, which starch, as extracted, has a viscosity onset temperature as determined by differential scanning calorimetry which is reduced by at least 5°C compared to starch extracted from equivalent, non-transformed plants. Preferably the viscosity onset temperature is reduced by at least 7°C. In another embodiment the invention provides altered starch extracted from a transformed potato plant or the progeny thereof, which starch, as extracted, has a viscosity onset temperature as determined by differential scanning calorimetry, of less than 60°C, preferably less than 55°C.

The starch defined above will typically also exhibit (as extracted) a reduced endotherm peak temperature, as determined by differential scanning calorimetry, compared to starch extracted from equivalent, non-transformed plants. Desirably the endotherm peak temperature will be reduced by at least 5°C and/or will be less than 59°C. The inventors have found that such properties as those defined above may be embodied in potato starch having a substantially normal amylose content (i.e. around 25 - 30% amylose).

Starch can be modified in various ways (e.g. chemical cross-linking, derivatisation, partial hydrolysis) after it has been extracted from a plant source, which modifications can affect the physical properties, especially the pasting properties, of the starch. Hence, use of the term "as extracted" is intended to signify that the starch is analysed without undergoing such modifications as can alter the pasting properties thereof.

"Equivalent, non-transformed" plants are those plants which have substantially identical genotypes to the plants of the invention, with the exception of the introduced nucleic acid sequence present in the transformed plants of the invention.

The invention will now be further described by way of illustrative example and with reference to the accompanying drawings, in which;

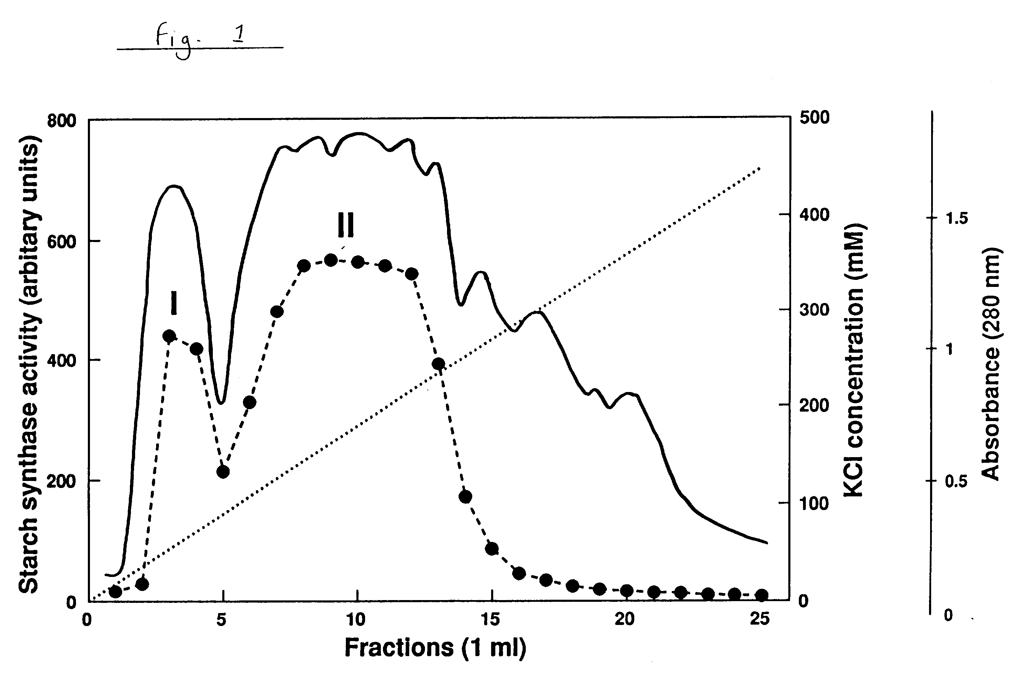

Figure 1 shows the elution profile of starch synthase from developing Desiree potato tubers on a first Mono Q™ anion-exchange column. Partially purified starch synthase, after DEAE-Sepharose and Blue Sepharose chromatography, was applied to a 1ml Mono Q™ column at pH 7.5. The enzyme was eluted with a 25ml gradient of 0-450 mM KCI at 0.5 ml.min⁻¹. Samples (20 µl) of each 1 ml fraction were assayed for starch synthase activity ( -●- ), and absorbance at 280 nm ( - );

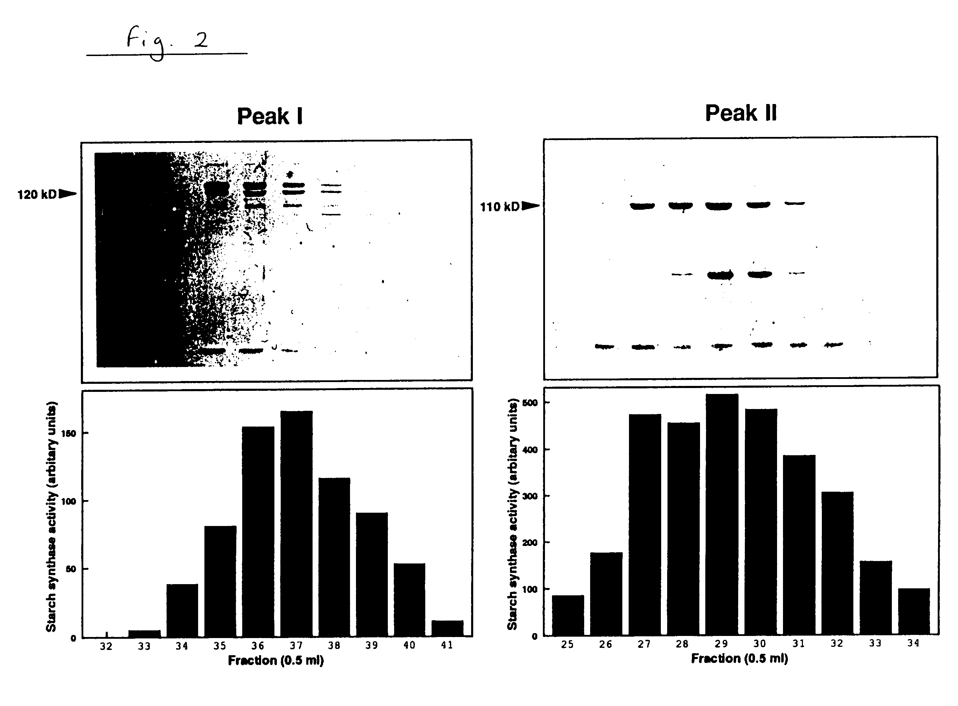

Figure 2 shows the activity and protein in fractions of purified starch synthase from a second Mono Q™ column of peak I and peak II. Top panels show SDS-PAGE of fractions containing starch synthase activity. Each track contains 10 µl of fraction. Bottom panels show starch synthase activity in 20 µl samples from each 0.5 ml fraction;

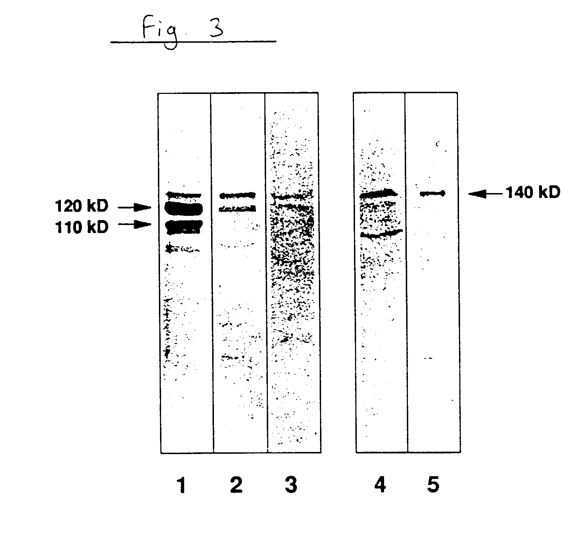

Figure 3 shows the cross-reaction of antiserum to SSS to the purified starch synthases from mature Estima tubers and to extracts, soluble and granule-bound, from mature Estima and developing Desiree tubers. Samples (10 µl of purified soluble starch synthase, 20 µl of partially purified soluble starch synthase, 20 µl soluble extract and 20 µl of supernatant from granule-bound proteins) were subjected to SDS-PAGE and blotted, and then the blots were probed with antiserum to SSS, 1/2500 dilution. (1) purified preparation of starch synthase proteins from mature Estima. (2) Partially purified soluble starch synthase from mature Estima tubers. (3) Starch-granule-bound proteins from mature Estima tubers. (4) Soluble extract from developing Desiree tubers. (5) Starch-granule-bound proteins from developing Desiree tubers. Sizes of proteins were estimated from molecular weight standards on the same gels, and are indicated in kDa;

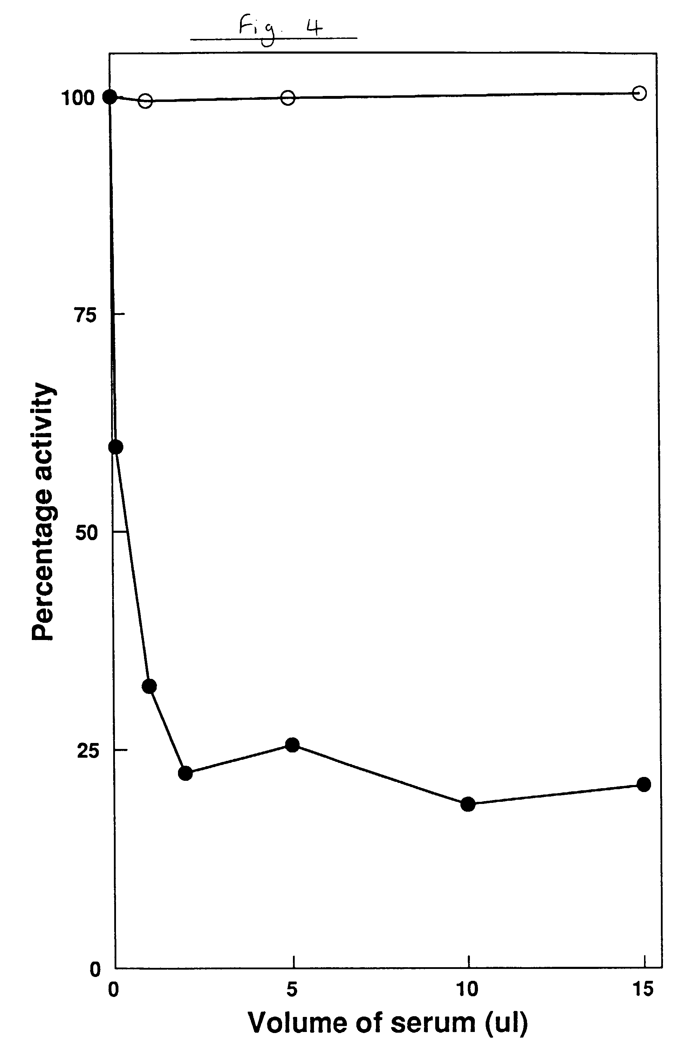

Figure 4 shows the immunoprecipitation of starch synthase activity in soluble extract from developing Desiree tubers with antiserum to SSS. Soluble extract was incubated with increasing volumes of pre-immune serum (○) and antiserum (●), as described in Materials and Methods (below). After centrifugation the supernatant was assayed for starch synthase activity. Starch synthase activity is expressed as a percentage of activity of incubations containing 20 g.L⁻¹ BSA in PBS. Values are from two separate experiments with the line joining the means;

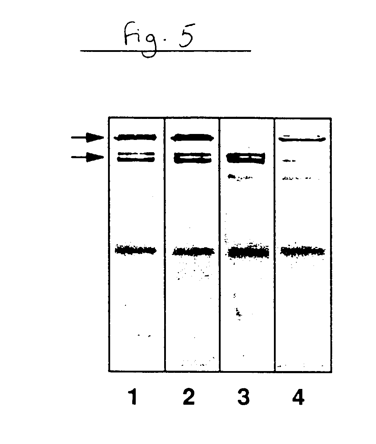

Figure 5 shows native polyacrylamide gel electrophoresis of soluble extract from developing Desiree tubers stained for starch synthase activity. Soluble extract was incubated (as described in Materials and Methods) in the presence of (1) 20 g.L⁻¹ BSA in PBS; (2) pre-immune serum, 1/1000 dilution; (3) antiserum to SSS, 1/1000 dilution; and (4) antiserum to the GBSS II from pea embryo. After centrifugation, the supernatant was mixed 5:1 with 2 g.L⁻¹ bromophenol blue in 500 ml.L⁻¹ glycerol and 40 µl was loaded onto the gel. The bands of starch synthase activity are indicated by arrows;
Figure 6 shows the DNA sequence of a cDNA clone for potato soluble starch synthase. The amino acid sequence of the encoded polypeptide is shown below in the single letter code. The ADP-glucose binding domain is boxed and the sequences identified by protein sequencing are underlined; and

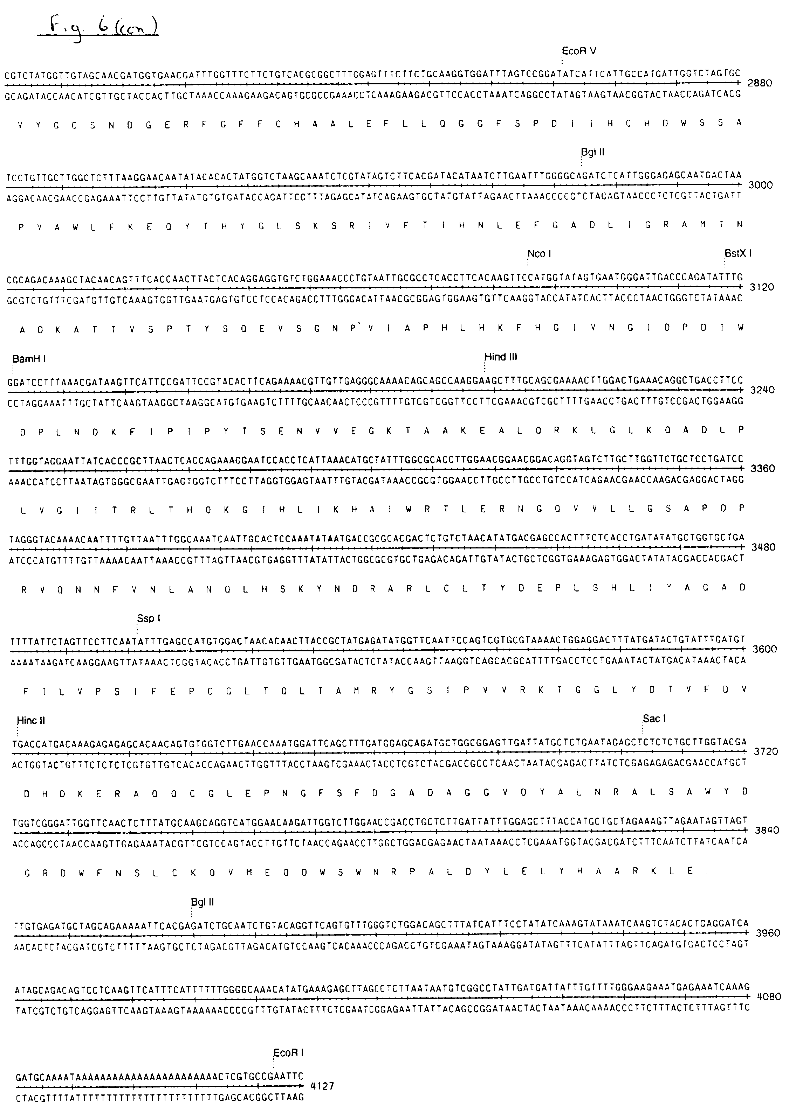

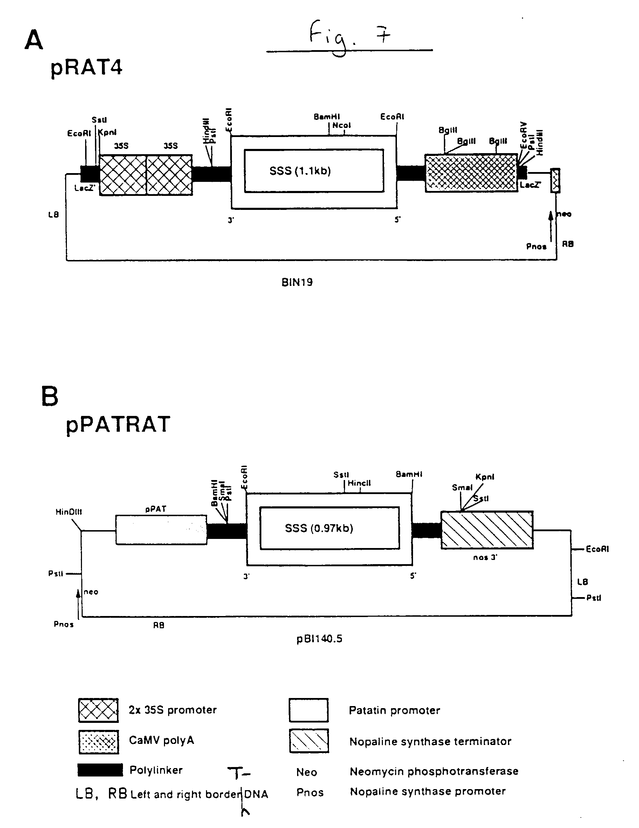

Figure 7 shows a schematic representation of A) plasmid pRAT4 and B) plasmid pPATRAT.

### EXAMPLES

### Example 1

In this example are presented data on the identification and purification to homogeneity of the major isoform of soluble starch synthase from potato tuber.

### MATERIALS AND METHODS

### Plant material.

Potato tubers (*Solanum tuberosum* L.) of cultivars Desiree (developing) or Estima (mature) were used. Desirée tubers were grown in pots of soil based compost (25 cm diameter) in a greenhouse with minimum temperature of 12°C and supplementary lighting in winter, and were freshly harvested prior to experiments from actively-growing plants. Estima tubers were bought locally.

### Purification of soluble starch synthase.

(A) Small scale. The extraction and subsequent purification were carried out at 4°C. Approximately 500g of Desiree potato tubers were chopped into small pieces and homogenised in an electric blender with 25g polyvinylpolypyrolidone (PVPP) and 500 ml of ice-cold medium A containing 100 mM Tris-HCl (pH 7.5), 10 mM ethylenediamine-tetraacetic acid (EDTA), 5 mM dithiothreitol (DTT), 1 g.L⁻¹ sodium metabisulphite, 0.5 mg.L⁻¹ leupeptin, 0.7 mg.L⁻¹ pepstatin A and 50 ml.L⁻¹ glycerol. The homogenate was passed through two layers of muslin and the filtrate was centrifuged at 10,000g for 10 min. The supernatant was brought to 40% saturation with powdered (NH₄)₂SO₄. The precipitate was collected by centrifugation (15,000g for 15 min), re-dissolved in a minimal volume of medium A and dialysed twice, each time against 1L of medium A for 1 h.

The dialysed extract was applied, at a flow rate of 4 ml.min⁻¹, to a column (5 cm internal diameter "i.d.", 10 cm long) of diethylaminoethyl (DEAE)-Sepharose Fast Flow™ (Pharmacia, Uppsala, Sweden), equilibrated with medium A. The column was washed with 500 ml of medium A followed by a 250-ml gradient of 0-1 M KCl in the same medium. Fractions of 10 ml were collected and assayed for starch synthase activity. The eight to ten fractions containing the highest activity were pooled and dialysed twice, each time against 1 L of medium B containing 50 mM Tris-HCl (pH 7.5), 1 mM EDTA, 1 mM DTT, 0.5 mg.L⁻¹ leupeptin, 0.7 mg.L⁻¹ pepstatin A and 50 ml.L⁻¹ glycerol for 1 h.

The dialysed extract was applied, at a flow rate of 1 ml.min⁻¹, to a column (1.6 cm i.d., 16 cm long), of Blue Sepharose, equilibrated with medium B. The column was washed with 100 ml medium B followed by a 100ml gradient of 0-1 M KCl in the same medium. Fractions of 0.5 ml were collected and assayed for starch synthase activity. The ten fractions with the highest activity were pooled and dialysed twice, each time against 1 L of medium B for 1 h.

The dialysed extract was applied, at a flow rate of 0.5 ml.min⁻¹, to a first 1-ml Mono Q™ column (Pharmacia), equilibrated with medium B. The column was washed with 25 ml of medium B, followed by a 25-ml gradient of 0-450 mM KCl in the same medium. Fractions of 0.5 ml were collected and assayed for starch synthase activity. The fractions from each of two peaks of starch synthase activity were pooled and purified separately as follows:

To the eluate of the first Mono Q™ column, an equal volume of 1 M sodium citrate in medium B was added. This was then applied, at a flow rate of 0.5 ml.min⁻¹, to a column (1.0 cm i.d., 4 cm long) of cyclohexa-amylose (CHA)-Sepharose (prepared according to Vretblad, 1974 FEBS Lett. 47, 86-89), equilibrated with 0.5 M sodium citrate in medium B. The column was washed with 20 ml medium B containing 0.5 M sodium citrate and the protein was eluted from the column with 30 ml of medium B containing no citrate. Fractions of 1 ml were collected and assayed for starch synthase activity. The fractions with starch synthase activity were pooled and dialysed overnight against 5 L of medium C containing 50 mM Tris-HCl (pH 8.0), 1 mM EDTA, 1 mM DTT, 0.5 mg.L⁻¹ Leupeptin, 0.7 mg.L⁻¹ pepstatin A and 50 ml.L⁻¹ glycerol.

The dialysed extract was applied, at a flow rate of 0.5 ml.min⁻¹, to a second 1ml Mono Q™ column equilibrated with medium C. The column was washed with 25 ml of medium C followed by a 25ml gradient of 0-450 mM KCl in the same medium. Fractions of 0.5 ml were collected and assayed for starch synthase activity.

### (B) Large scale.

The procedures were as described above, with the following modifications. Five kg of Estima potato tubers were homogenised in 5 L medium A containing 250g PVPP, filtered through two layers of muslin and centrifuged at 10,000g for 10 min. Polyethylene glycol (PEG) 6000 at a concentration of 500 g.L⁻¹ in medium A was slowly added to the supernatant until the concentration of PEG was 100 g.L⁻¹. The precipitate was collected by centrifugation (15,000g for 20 min) and re-dissolved in a minimal volume of medium A.

The extract was mixed gently for 1 h with 900ml slurry of DEAE-Sepharose which had been equilibrated with medium A, then filtered and the filtrate discarded. The DEAE-Sepharose was washed with 2 L medium A then incubated for 1 hr in 500 ml medium A containing 400 mM KCl, filtered and washed with a further 500 ml medium A containing 400 mM KCl. The filtrates were combined and brought to 50% saturation with powdered (NH₄)₂SO₄. The precipitated proteins were collected by centrifugation (15,000g for 15 min), re-dissolved in a minimal volume of medium B and dialysed overnight against 5 L of medium B.

The dialysed sample was applied, at a flow rate of 2 ml.min⁻¹, to a Blue Sepharose column (5 cm i.d., 15 cm long) which had been equilibrated with medium B. The column was washed with 300 ml medium B followed by a 600ml gradient of 0-1 M KCl in the same medium, at a flow rate of 5 ml.min⁻¹. Fractions of 15 ml were collected and assayed for starch synthase activity. The ten fractions with the highest starch synthase activity were pooled and dialysed ovemight against 5 L medium B.

The dialysed eluate was applied to a first 1ml Mono Q™ column, equilibrated with medium B, as described above, except that all the fractions containing starch synthase activity were pooled together.

The Mono Q™ eluate was applied to a CHA-Sepharose column as described above, except that the column was 1.0 cm i.d., 20 cm long. The column was washed with 50 ml medium B containing 0.5 M sodium citrate and eluted with 80 ml medium B without citrate. The fractions with starch synthase activity were pooled and dialysed ovemight against 5 L of medium C.

The dialysed extract was applied to a second 1ml Mono Q™ column equilibrated with medium C, as described above. Fractions containing starch synthase activity were stored at -20°C.

### Preparation of antibody.

The fractions containing starch synthase activity from 5 large-scale purifications were run on preparative sodium dodecyl sulphate (SDS)-polyacrylamide gels (as described below). The gel slices containing starch synthase proteins were electroeluted and the proteins dialysed against water, then freeze-dried. Protein (50 µg) was re-dissolved in 250 µl of phosphate-buffered saline (PBS), mixed with 250 µl Freund's complete adjuvant, and injected intramuscularly into a rat. Subsequent injections were of 75 µg protein dissolved in 250 µl PBS mixed with 250 µl Freund's incomplete adjuvant and were repeated at 14-day intervals. Serum was collected from 14 days after the third injection.

### Assay of soluble starch synthase activity.

Soluble starch synthase activity was measured using the resin method as described in Jenner *et al.* (1994).

### Preparation of crude soluble potato tuber extract.

Samples (0.5-2.0 g fresh weight) from either developing Desiree or mature Estima potato tuber were homogenised in 4 volumes of 50 mM Tris-HCl (pH 7.5), 1 mM EDTA, 1 mM DTT, 1 g.L⁻¹ sodium metabisulphite, 0.5 mg.L⁻¹ leupeptin, 0.7 mg.L⁻¹ pepstatin A and 50 ml.L⁻¹ glycerol, then centrifuged at 10,000g for 10 min. The supernatant is referred to as "soluble extract".

### Partial purification of soluble starch synthase activity.

Crude soluble potato extract from mature Estima tubers (5-10 g fresh weight) was dialysed twice, each time against 1 L of buffer B for 1 hr. The dialysed extract was applied to a lml Mono Q™ column, equilibrated with medium B, as described above and the peak fraction of starch synthase activity (referred to as "partially purified soluble starch synthase") was stored at -20°C.

### SDS-polyacrylamide gel electrophoresis (PAGE) and immunoblotting.

Protein samples were dialysed against distilled water then mixed 1:1 with double-strength sample buffer (Laemmli, U.K. (1970). Nature 227, 680-685) and boiled for 2 min immediately prior to application to gels. For granule-bound proteins, starch granules were washed twice in 20 g.L⁻¹ SDS at room temperature, boiled for 3 min at 100 mg.ml⁻¹ in sample buffer (Laemmli, U.K. (1970). Nature 227, 680-685) and then centrifuged at 10,000g for 10 min. The supernatant was applied to the gel.

Gels (10.2 cm long, 7.3 cm wide, 0.75 mm thick) were 75 g.L⁻¹ acrylamide (37.5:1 w/w acrylamide:bis-acrylamide) and 1 g.L⁻¹ SDS and were run according to Laemmli (1970). Immunoblots were prepared and developed according to Bhattacharyya *et al.,* (1990) Cell 60, 115-122. The nitrocellulose filters were either incubated with crude rat serum followed by alkaline phosphatase-congugated goat anti-rat antiserum (Sigma, Poole, Dorset, UK) or the immunoglobulin fraction of rabbit serum to GBSS II from pea embryo (Smith (1990) Planta 182, 599-604), followed by alkaline phosphatase-conjugated goat anti-rabbit antiserum (Sigma).

### Native polyacrylamide gel electrophoresis.

Gels (dimension as above, except lmm thick) of 90 g.L⁻¹ acrylamide (37.5:1 w/w acrylamide:bis-acrylamide) were cast in 400 mM Tris-HCl (pH 8.6), 100 ml.L⁻¹ glycerol, 8 g.L⁻¹ glycogen and polymerised with 0.4 g.L⁻¹ ammonium persulphate and 0.2 ml.L⁻¹ N,N,N',N'-tetramethylethylenediamine (TEMED) and were overlaid with a stacking gel of 53 g.L⁻¹ acrylamide (37.5:1 w/w acrylamide:bis-acrylamide) cast in 155 mM Tris-HCl (pH 6.8), 98 ml.L⁻¹ glycerol, polymerised with 0.5 g.L⁻¹ ammonium persulphate and 0.2 ml.L⁻¹ TEMED. Soluble extracts were mixed 5:1 with 2 g.L⁻¹ bromophenol blue in 500 ml.L⁻¹ glycerol immediately prior to loading. Gels were run at 4°C, at 175 mV in 190 mM glycine, 25 mM Tris.

The gel was assayed for starch synthase activity as follows. The gel was washed twice, each time for 10 min in 20 ml 100 mM Bicine, 0.5 M sodium citrate (pH 8.5), 0.5 M EDTA and 100 ml.L⁻¹ glycerol at 4°C. The gel was incubated at room temperature for 20 hrs by gently shaking in wash medium containing 12 mM ADPG and 2 mM DTT. The buffer was removed and 1 ml of Lugol's iodine solution (3.3 g.L⁻¹ I₂ and 6.7 g.L⁻¹ KI, acidified with a few drops of 2M HCl) was added. After colour development, the gel was washed and stored in 70 ml.L⁻¹ acetic acid.

### Immunoprecipitation.

Soluble extracts (100µl) were incubated with 0-20µl rat serum or 20µl of the immunoglobulin fraction of rabbit serum to GBSS II from pea embryo (Smith (1990) Planta 182, 599-604) for 1.5 h at room temperature on a rotating table. To the extract containing rat serum, 20µl polyclonal antiserum to rat IgG at 2.5 g.L⁻¹ specific antibody (Sigma) was added and incubated for a further 0.5 h. To both extracts, 50 µl Protein A-Sepharose at 60 g.L⁻¹ in 50 mM Tris-HCl (pH 7.5) was added and then incubated for 0.5 h, followed by centrifugation at 10,000g for 10 min. The supernatants were assayed for starch synthase activity. Controls contained bovine serum albumin at 20 g.L⁻¹ in PBS in place of serum.

### Isolation of starch granules.

Purified starch was prepared from potato tubers as described by Edwards *et al.* (1995).

### Measurement of protein.

Protein was assayed using the BioRad Protein Assay Dye Reagent (BioRad Munchen, Germany) with a standard curve of bovine serum albumin.

### RESULTS

### Purification of soluble starch synthases.

The soluble starch synthase activity from developing tubers of Desirée and mature tubers of Estima eluted from both DEAE-Sepharose and Blue Sepharose columns as a single peak of activity. However, subsequent chromatography on a Mono Q™ column at pH 7.5 separated two major peaks of starch synthase activity, designated peak I and peak II according to their elution order from the column (Figure 1). These two peaks of starch synthase activity were then purified separately by cyclohexa-amylose and Mono Q™ chromatography. A typical purification from developing Desiree tubers is shown in Table 1. The specific activity of peak I was 5.1 µmol.(mg protein)⁻¹.min⁻¹, a purification of 400-fold relative to the initial supernatant. The specific activity of peak II was 8.8 µmol.(mg protein)¹.min⁻¹, a purification of 700-fold relative to the initial supernatant (Table 1).

Table 1 shows the purification of soluble starch synthase from developing potato tubers of Desiree. Fractions were prepared as described above. The values shown in the table are from a typical purification.

**TABLE 1**

| FRACTION | TOTAL ACTIVITY (µmol glucose incorporated min⁻¹) | ACTIVITY RECOVERED (%) | TOTAL PROTEIN (mg) | SPECIFIC ACTIVITY (µmol glucose incorporated min⁻¹.mg protein⁻¹) |
|---|---|---|---|---|
| Initial Supernatant | 28.9 | 100 | 2210.6 | 0.013 |
| 0 to 40% (NH₄)₂SO₄ | 17.1 | 61.0 | 1018.6 | 0.017 |
| DEAE-Sepharose | 7.51 | 26.8 | 45.1 | 0.166 |
| Blue-Sepharose | 4.59 | 16.4 | 10.4 | 0.441 |

| Peak I | | | | |
|---|---|---|---|---|
| Mono Q (pH 7.5) | 0.95 | 3.4 | 1.70 | 0.56 |
| Cyclohexa-amylose | 0.53 | 1.9 | 0.20 | 2.65 |
| Mono Q (pH 8.0) | 0.15 | 0.5 | 0.03 | 5.13 |

| Peak II | | | | |
|---|---|---|---|---|
| Mono Q (pH 7.5) | 2.45 | 8.8 | 2.90 | 0.85 |
| Cyclohexa-amylose | 2.27 | 8.1 | 0.40 | 5.67 |
| Mono Q (pH 8.0) | 0.26 | 0.9 | 0.03 | 0.84 |

**TABLE 2**

| INCUBATION | INHIBITION OF STARCH SYNTHASE ACTIVITY (%) |
|---|---|
| Pre-immune serum | 0.3 ± 0.9 |
| Antiserum to potato SSS | 74 ± 4 |
| Antiserum to pea GBSS II | 9 ± 4 |
| Antiserum to potato SSS + pea GBSS II | 80 ± 8 |

Table 2 shows the immunoprecipitation of starch synthase activity in soluble extract from developing Desiree tubers. Soluble extract was incubated in the presence of antiserum (1/10 dilution of rat antiserum; or 1/5 dilution of the immunoglobulin fraction of rabbit serum to GBSS II from pea embryo; or 1/10 dilution of rat antiserum plus 1/5 dilution of the immunoglobulin fraction of rabbit serum to GBSS II), as described in Material and Methods. After centrifugation the supernatant was assayed for starch synthase activity. Values are percentage inhibition relative to controls in which BSA at 20 g.l⁻¹ in PBS was substituted for serum. The values are the mean of four experiments ± standard error.

Sodium dodecyl sulphate polyacrylamide gel electrophoresis (SDS-PAGE) of the fractions from the second Mono Q™ column for peak I showed that the distribution of a protein of 120 kDa matched the distribution of the starch synthase activity (Figure 2). Further chromatography on Mono Q™ did not eliminate contaminating proteins. SDS-PAGE of the fractions from the final Mono Q™ column for peak II showed that the distribution of the major protein of 110 kDa matched the starch synthase activity (Figure 2).

Antibodies raised to the 59 kDa starch-granule-bound protein (the GBSS I isoform) from pea embryo did not recognise any proteins from either peak I or peak II. Antibodies raised to the 77 kD GBSS II from pea embryo very weakly recognised the 120 and 110 kDa proteins from peak I and II respectively (data not shown).

### Preparation of antibody.

In order to obtain sufficient protein for preparation of an antibody, peaks I and II from mature Estima tubers were combined and purified together in large-scale preparations (referred to as "soluble starch synthase", SSS). Both the 120- and 110 kDa proteins were excised and eluted from gels of the purified preparations and were injected into the same rat.

The antiserum to the SSS was used to probe blots of extracts from mature Estima and developing Desiree tubers (Figure 3). On all of the immunoblots, the pre-immune serum did not cross-react with any of the proteins. On immunoblots of the gels of the purified preparation of soluble starch synthase from mature Estima tubers, the antiserum recognised strongly the two proteins to which it was raised. The antiserum also recognised a minor protein of 140 kDa. On immunoblots of gels of partially purified soluble starch synthase from mature Estima tubers, the antiserum recognised proteins of 140 kDa and 120 kDa. On immunoblots of gels of starch-granule-bound proteins from mature Estima tubers, the antiserum recognised a protein of 140 kDa. There were some faint indications that a 120 kDa protein on the starch was also recognised. A protein of 140 kDa was recognised by the antiserum both in the soluble extracts and on starch granules of developing tubers of Desiree. The 120 kDa protein was very weakly detectable in soluble extracts from these tubers, which also contained a lower molecular weight protein (approximately 100 kDa) recognised by the antiserum. This protein is not GBSS II since the rat antiserum did not recognise GBSS II on starch granules (data not shown).

### Immunoprecipitation of starch synthase activity.

To discover whether the proteins recognised by the antiserum to SSS represent the major soluble starch synthases, the antiserum was used in immunoprecipitation experiments with soluble extracts from developing Desiree tubers.

Incubation of soluble extract with pre-immune serum from the rat did not affect soluble starch synthase activity, but the antiserum to SSS precipitated starch synthase activity (Figure 4). The maximum inhibition of starch synthase activity was approximately 75 % which was achieved by incubating with volumes greater than 2µl of antiserum. A small proportion of the remaining starch synthase activity can be accounted for by GBSS II (Table 2). When soluble extract is incubated with antiserum raised to GBSS II from pea embryo (which recognises GBSS II in potato, Edwards *et al.,* 1995), approximately 9% of the starch synthase activity is inhibited. When the potato extract is mixed with both antibodies, the starch synthase activity is reduced by approximately 80%.

Native polyacrylamide gel electrophoresis of soluble extracts of developing Desirée tubers revealed two major groups of bands which had starch synthase activity (Figure 5). We have previously shown through antisense and immunoprecipitation experiments that the lower group of bands is attributable to GBSS II. Tubers in which GBSS II protein has been severely reduced by antisense transformation lack the lower group of bands. When the soluble extract was immunoprecipitated with antiserum to GBSS II from pea embryo and the supernatant subjected to native PAGE, the lower bands were missing (Edwards *et al.,* 1995 cited previously). Immunoprecipitation of soluble extract from developing Desiree tubers with rat antiserum to SSS shows that the upper group of bands is attributable to these starch synthases. When the supernatant from the immunoprecipitation experiment was subjected to native PAGE, the upper group of bands was missing but the lower group was unaffected. The pre-immune serum from rat had no effect on the bands of starch synthase activity.

### DISCUSSION

The inventors have purified two proteins with starch synthase activity from the soluble fraction from mature Estima tubers, with molecular weights of 110 and 120 kDa respectively. Immunoblots show that the antiserum raised to these purified proteins (soluble starch synthases, SSS) recognises the proteins to which it was raised, and that it also recognises a higher molecular weight protein of 140 kDa in the purified preparation (Figure 3). The 140 kDa protein is in soluble extracts and on starch granules of both mature Estima and developing Desiree tubers, whereas the 120 kDa protein is either barely or not detectable in tubers, and the 110 kDa protein is not detectable at all. This strongly suggests that the two starch synthase proteins to which antibodies have been raised may both be active breakdown products of the larger 140 kDa protein, although the 140 kDa polypeptide might simply be an immunologically cross-reactive entity. Although most of the breakdown undoubtedly occurs during purification (despite the purification being carried out at 4°C and with the inclusion of PVPP and protease inhibitors), some of the breakdown may also occur *in vivo.* Breakdown of enzymes *in vivo* has been observed during the purification of starch branching enzyme from potato tubers. Using fresh harvested tubers for the purification resulted in a predominately high molecular weight starch branching enzyme being isolated, but when stored tubers were used, a wide range of molecular weight proteins were isolated (Blennow & Johansson, 1991 Phytochem. 30, 437-444).

The fact that the antiserum to SSS recognises the 140 kDa protein in both Estima and Desiree tubers suggests that there is no difference between the two cultivars in their major starch synthases, and vindicates the use of these two different cultivars in the work reported herein. The occurrence of a 100 kDa protein antigenically related to the 140 kDa protein in Desiree tubers is interesting, and at present it is not known what that protein may be. Its absence from Estima tubers could reflect the fact that these cultivars were stored rather than developing tubers, or could represent a difference between cultivars.

Specific activities of the purified soluble starch synthases from potato tuber are comparable with or greater than these of soluble starch synthases from other storage organs. Purification to homogeneity of isoforms of soluble starch synthase resulted in specific activities of 16 µmol.(mg protein)⁻¹.min⁻¹ from pea embryo (Denyer & Smith 1992 cited previously), 14 µmol.(mg protein)⁻¹ min⁻¹ from wheat (Denyer *et al.,* 1995 cited previously) and 9 µmol.(mg protein)⁻¹.min from maize (Mu *et al.* (1994) Plant J. 6, 151-159). The specific activity of the soluble starch synthase reported in this application is 7- to 300-fold higher than that of the partial purifications of soluble starch synthase activity from potato tuber reported by Hawker *et al.,* 1972 Phytochem. *11*, 1278-1293: 0.64 µmol.(mg protein)⁻¹.min⁻¹), Baba *et al.*(1990) Phytochem. 29, 719-723: 0.03 µmol.(mg protein)⁻¹.min⁻¹) and Ponstein (1.35 and 0.91 µmol.(mg protein)⁻¹.min⁻¹).

The immunoprecipitation experiments also suggest that the purified proteins are the major soluble starch synthases in potato tuber, or are products directly derived from such synthases. The antiserum raised against the soluble starch synthase from potato precipitates 75 % of the total soluble starch synthase activity in crude extract (Figure 5). The remainder of the activity is partly due to GBSS II (Table 2), but the possibility of further minor isoforms cannot be ruled out.

The purified soluble starch synthase is likely to represent a novel class of starch synthase. It is not related to the major soluble starch synthase in pea embryo (GBSS II), which is clearly related to the minor, soluble 92 kDa GBSS II in potato. The soluble starch synthase is only very weakly recognised by the antibody raised to GBSS II from pea. It is not related to the GBSS I proteins either: the starch synthase from potato tuber is not recognised by the antibody raised to GBSS I from pea embryo. These results reinforce the view that storage organs differ profoundly in the nature and number of active isoforms of starch synthase (Smith *et al.,* 1995 Plant Physiol. *107,* 1; Edwards *et al.,* and Denyer *et al.,* both cited previously).

### Example 2

### ISOLATION OF A cDNA CLONE FOR SOLUBLE STARCH SYNTHASE FROM POTATO TUBERS

The antiserum raised to the purified starch synthase protein from Estima tubers was used for immunoscreening of a λgt 11 library (provided by C Grierson, John Innes Centre, Norwich) containing cDNA inserts with *Eco*RI linkers, constructed from developing Estima tuber poly[A] RNA.

Approximately 1.5 x 10⁶ plaque-forming units were probed with the antiserum at a dilution of 1/1000. The second antibody was an anti-rat immunoglobulin linked to horseradish peroxidase (Amersham International, Amersham, UK). Two positive clones were isolated. These were both 1.1 kb in length and contained poly(A) tracts at their 3' ends. One of these was cloned into the EcoRI site of pBluescript SK + to give plasmid pRAT2. A 5' *Eco*Rl*-ECOR*V fragment from this clone was used as a probe on the λgt11 library. Filters were washed in 0.1 x SSC (1 x SSC is 0.15 M NaCl, 0.015 M sodium citrate), 0.5 g L⁻¹ SDS at 65°C. Seven clones of 1.3, 1.53, 1.75, 1.88, 2.15, 2.21, and 2.4 kb were isolated. The longest clone was subcloned as an *Eco*RI fragment into pBluescript SK+ to give plasmid pRAT20. A 600-bp 5' fragment from pRAT20 was used to probe a random primed λgt11 library prepared from cDNA of developing tubers. Three positive clones were isolated. The longest was 2.3 kb and was subcloned as an *Eco*RI fragment into pBluescript SK+ to give pRAT24.

The 2.3 and 2.4kb partial clones overlapped. The full-length composite cDNA was 4.127kb. The DNA sequence of the full length cDNA, and the predicted polypeptide sequence, are shown in Figure 6. DNA sequences were determined according to Sanger *et al.* (1977) by using Sequenase™ (United States Biochemical). Sequence data were analysed using the Genetics Computer Group (Madison, WI) computer program (Devereux *et al.* 1984 Nucl. Acids Res. 12, 387-395).

To check the identity of the cDNA, the amino acid sequence it predicted was compared with amino acid sequences of two peptides obtained by digestion with endoproteinase Lys-C of the 110-kD protein purified from tubers of cultivar Estima. The peptide sequences FIPIPYTSENVVEGK (Seq. ID No. 1) and HIPVFGG (Seq. ID No. 2) corresponded precisely to predicted sequences from the clone. Attempts to obtain N-terminal amino acid sequence of the purified proteins for comparison with the sequence predicted from the cDNA clone were unsuccessful.

On RNA gel blots of poly(A)⁺RNA from developing tubers, a partial cDNA clone recognised a single transcript of ∼4 kb. This size is considerably greater than those of the transcripts for GBSSI and GBSSII and is consistent with the transcript encoding a protein in the range of 110 to 140 kD.

The deduced amino acid sequence of the soluble starch synthase revealed a protein of 1230 amino acids and a predicted size of 139 kD (Figure 6). At the N terminus was a sequence of ∼60 amino acids rich in serine and basic residues and low in acidic residues, which is typical of a chloroplast transit peptide. Based on the consensus of Gavel and von Heinje (1990 FEBS Lett. 261, 455-458), the most likely cleavage site would be between amino acids 60 (Cys) and 61 (Ala), because the serine-rich region ends before this point. Cleavage in this region would give a mature protein of ∼132 kD. The structure is somewhat similar to that of GBSSII in that it contains a C-terminal region homologous with starch synthases and bacterial glycogen synthases and an N-terminal extension. The N-terminal extension shows little sequence similarity to the N-terminal extensions of GBSSII from pea or potato (in turn, they show little similarity to each other; Edwards *et al.,* (1995) Plant J. 8, 283-294) or to any other sequence in the data bases. The N-terminal domain resembles those of pea and potato GBSSII in that it shows considerable predicted flexibility (Chou-Fasman algorithm; see Dry *et al.,* (1992) Plant J. 2, 193-202); all these extensions may therefore serve similar roles. At the C-terminal end of the N-terminal extension of the soluble starch synthase are two proline residues; multiple proline residues have been noted previously at the C-terminal ends of N-terminal extensions of both starch synthases and starch-branching enzymes (Dry *et al.,* (1992) Plant J. 2, 193-202; Burton *et al.,* 1995).

The roles of these N-terminal extensions are not known, but it seems likely that they are involved in determining properties such as interaction with starch polymers rather than contributing to basic catalytic properties. The C-terminal region from amino acid 780 to the end shows greatest similarity to glycogen synthases from bacteria, although there is also similarity to other starch synthases from plants. The KTGG motif close to the N terminus of this region beginning with position 794 is conserved (KVGGL). This domain is thought to be involved in ADP/ADP-glucose binding (Furukawa *et al.,* 1990 J. Biol. Chem. 265, 2086-2090). Interestingly, a second domain with a similar structure is also conserved in the C termini of all bacterial glycogen synthases and starch synthases (including the motif beginning at position 1143, T/V GGLXDT I/V); this may represent a second domain involved in ADP/ADP-glucose binding. The whole region around this second domain is widely conserved among α-1,4-glucosyltransferases, indicating close involvement with the catalytic process.

Over the rest of the soluble starch synthase protein, there are several other domains showing conservation between different starch synthases. However, it also shows some notable gaps in its sequence when aligned with GBSSI and GBSII, for example, between amino acids 828 to 829 (13 amino acids), 894 to 895 (10 amino acids), and 944 to 945 (35 amino acids). These regions may confer specific properties on GBSSI and GBSII compared with the soluble synthase.

### Example 3

### POTATO TRANSFORMATION

Binary vectors containing a partial cDNA for soluble starch synthase ("SSS") in the antisense orientation, under the control of a) the double 35S promoter or b) the patatin promoter have been constructed. The 2 x 35S construct is detailed below.

### Construction of Antisense Binary Vector

The 1.1-kb *Pst-Eco*RV fragment from pRAT2 encoding the 3' end of the soluble starch synthase was subcloned in an antisense orientation between the cauliflower mosaic virus double 35S promoter and cauliflower mosaic virus terminator *(Pst*I*-Sma*I*)* in pJIT60 (Guerineau and Mullineaux, 1993 "Plant transformation and expression vectors". In Plant Molecular Biology Labfax R.R.D. Croy, Ed. (Oxford, UK BIOS Scientific Publishers) p121-148), producing pRAT3. The *Xho*I-partial *Sst*I fragment from pRAT3, encompassing the promoter, antisense cDNA, and terminator, was ligated between the *Sal*l*-Sst*l sites of the plant transformation vector pBIN19 (Bevan, 1984 Nucl. Acids Res. 12, 8711-8721), resulting in plasmid pRAT4. This plasmid is illustrated schematically in Figure 7, which Figure also shows the plasmid pPATRAT comprising the patatin promoter.

### Transformation of Potato

Binary plasmid pRAT4 was introduced into *Agrobacterium tumefaciens* by the freeze-thaw method of An *et al.,* (1988 Binary Vectors. In Plant Molecular Biology Manual A3. Eds, Gelvin S.B. and Schilperoort R.A. ppl-19). Preparation of Agrobacterium inoculum carrying the antisense construct, inoculation of tuber discs of potato cultivar Desiree, regeneration of shoots, and rooting of shoots were as described by Edwards *et al.* (1995 Plant J. 8, 283-294).

Thirteen independently transformed plants and four independent control plants (transformed with the vector alone) were transferred to a soil-based compost and allowed to develop tubers. The presence of the SSS antisense construct was confirmed by DNA gel blotting (data not shown). Six of the transgenic plants had levels of SSS transcript indistinguishable from those of the control plants on RNA gel blots. However, seven independent transformants (named 1, 2, 9, 18, 19, 25 and 26) had strongly reduced or undetectable levels of SSS transcript. The loss or reduction of detectable transcript was specific for SSS, and there was little variation in the level of transcript for GBSSI among the plants studied (data shown in Marshall *et al.,* 1996 The Plant Cell 8, 1121-1135).

Tubers of the transformants with unaltered levels of SSS transcript had soluble starch synthase activities that were indistinguishable from those of the control plants and from values typical of those obtained from developing Desiree tubers in general (Edwards *et al.* (1995) Plant J. 8, 283-294 1995). Tubers of the seven transformants with reduced or undetectable levels of SSS transcript had significantly reduced activities, and three plants displayed activities that were 30% or less of the average value of the control plants. Table 3 shows that the observed reductions in soluble starch synthase activity were reproducible from one tuber to another. They were also reproducible through tuber development.

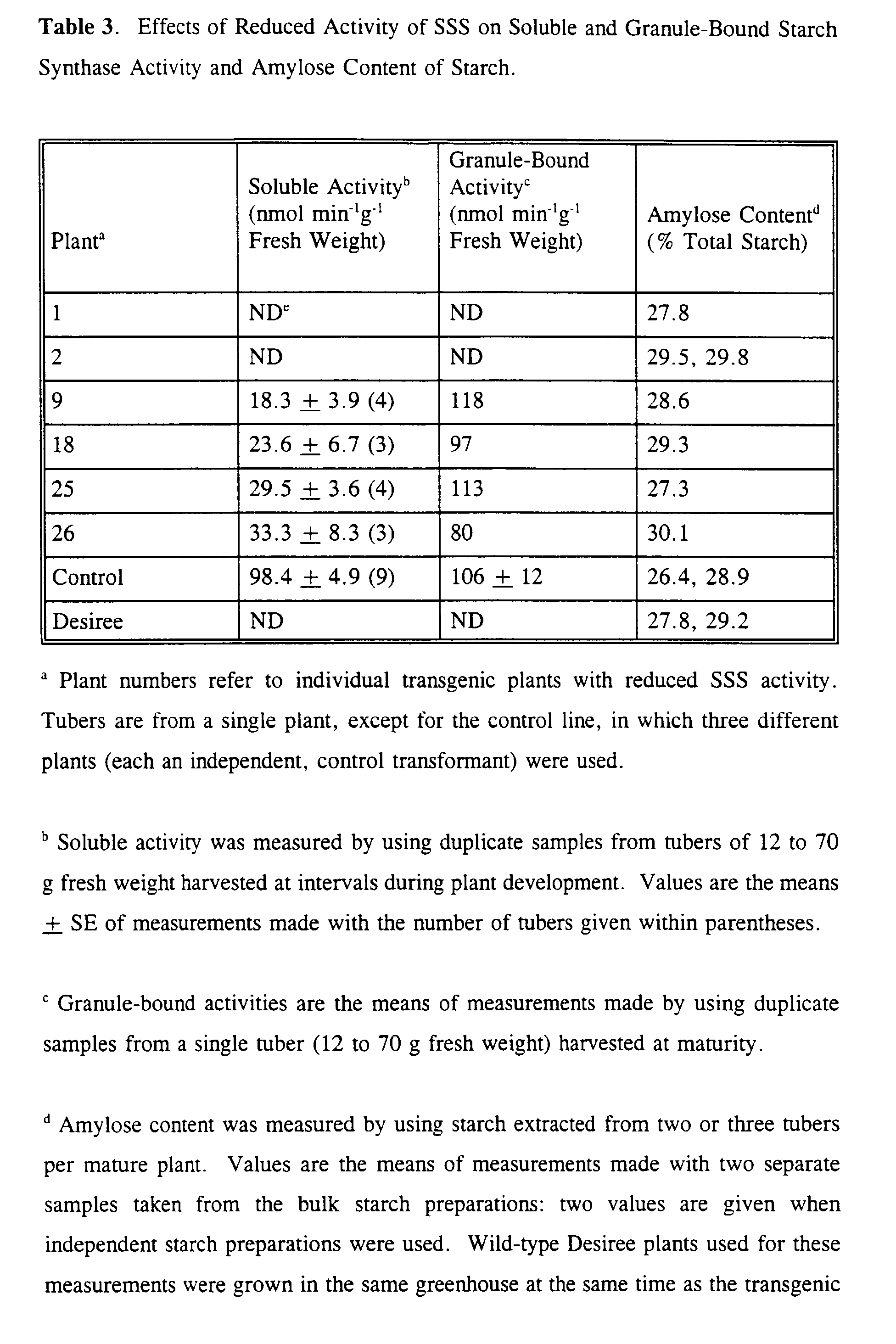

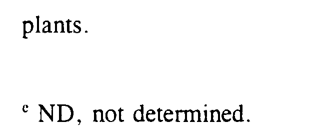

### Reductions in Starch Synthase Activity Are Specifically Due to Loss of SSS

To discover whether the reductions in activity were specifically attributable to loss of SSS, two sorts of experiments were undertaken. First, isoforms were visualised on native gels of crude, soluble extracts of transformed tubers. The group of bands attributable to SSS was present in extracts from control plants and from all six of the transformants with soluble starch synthase activities comparable with control activities. It was absent from extracts of all seven transformants with reduced starch synthase activities. Other groups of bands on the gels, including those attributable to GBSSII, were present in all extracts (data shown in Marshall *et al.,* 1996).

Second, crude, soluble extracts from a plant with strongly reduced activity were incubated with the antiserum raised against SSS. The antiserum inhibited activity by 16%, compared with 75 % inhibition in extracts of untransformed tubers of cultivar Desiree (Table 2).

Loss of starch synthase activity from the soluble fraction in transgenic tubers was accompanied by dramatic reductions in the amount of the 140-kD protein recognised by the antiserum in soluble and granule-bound fractions of the tuber. The protein was not detected, or detected only very weakly relative to controls, on immunoblots of these fractions from tubers of the six transgenic lines with the largest reductions in starch synthase activity. In contrast, the soluble protein of 105 kD also recognised by the antiserum was present in equal amounts in all lines examined (data shown in Marshall *et al*., 1996).

### Reduction in SSS Activity Alters Granule Shape but Has Little Effect on Starch and Amylose Content

Tubers of the seven transformants with reduced activities of soluble starch synthase had starch granules with strikingly altered morphology. Two types of granule were present: simple granules with deep, often T-shaped cracks centered on the hilum, and granules that appeared to be large clusters of tiny, spherical granules. A range of different sizes of both types of granule was present in tubers at various developmental stages (data not shown).

In spite of the alteration in granule morphology, tubers of transformants with reduced activity of SSS were indistinguishable from control tubers with respect to total starch content. This was true of both developing tubers and tubers of mature plants on which the haulm was senescent. The starch of these plants also displayed no significant alteration in amylose content (Table 3).

### Reduction in SSS Activity Does Not Affect Other Isoforms of Starch Synthase

It was thought possible that the reduction in SSS in transformed tubers may have secondary effects on other isoforms of starch synthase. Any alterations in other isoforms could seriously affect deductions about the importance and role of SSS and might prevent alteration of starch properties in transformed plants. Effects of the reduction in SSS on GBSSI were assessed by measuring granule-bound starch synthase activity in crude extracts of tubers and examining gels of granule-bound proteins. There was no difference in granule-bound activity between control plants and those in which soluble starch synthase acitivity was reduced (Table 3). More than 95 % of the starch synthase activity of intact starch granules of wild-type potatoes is attributable to GBSSI (Edwards *et al.* (1995) Plant J. 8, 283-294 1995). Reductions in SSS also had no obvious effect on the amount of GBSSI protein bound to starch granules (data not shown).

Effects of reductions in SSS on GBSSII were assessed in three ways. First, amounts of GBSSII protein in the soluble and granule-bound fractions of the tuber were visualised by immunoblotting. There were no obvious differences between control plants and those in which SSS was reduced.

Second, as described above, GBSSII was visualised on native gels of crude, soluble extracts stained for starch synthase activity. Again, there were no marked or consistent differences between control plants and those in which SSS was reduced.

Third, immunoprecipitation experiments were used to assess the proportion of the residual activity attributable to GBSSII in tubers in which SSS was reduced. The antiserum raised against GBSSII of pea embryos, which recognises GBSSII of potatoes (Edwards *et al.* 1995 cited above), inhibited ∼40% of the activity in tubers in which soluble starch synthase activity was reduced by ∼80% (line 9) compared with 9% in control and wild-type tubers (Table 2). Using these figures and starch synthase activities from Table 3, the activity attributable to GBSSII is 7.3 nmol min⁻¹g⁻¹ fresh weight in line 9 and 8.8 nmol min⁻¹g⁻¹ fresh weight in control tubers. This indicates that the reduction in SSS has little effect on the soluble activity of GBSSII.

### Example 4

### Detailed analysis of starch from tubers obtained from transformed Potato plants

Despite the results of crude analysis described in Example 3, indicating that the starch from transformed plants was essentially unaltered, it was decided to perform more detailed analysis of the starch, by Differential Scanning Calorimetry and Viscoamylograph. Analysis was performed as described in WO 95/26407 and WO 96/34968.

Surprisingly it was found that certain physical properties of the starch were consistently significantly altered. In particular, it was found that the viscosity onset temperature was significantly reduced compared to starch obtained from equivalent control plants which did not contain the SSS antisense construct. The results are shown in Table 4.

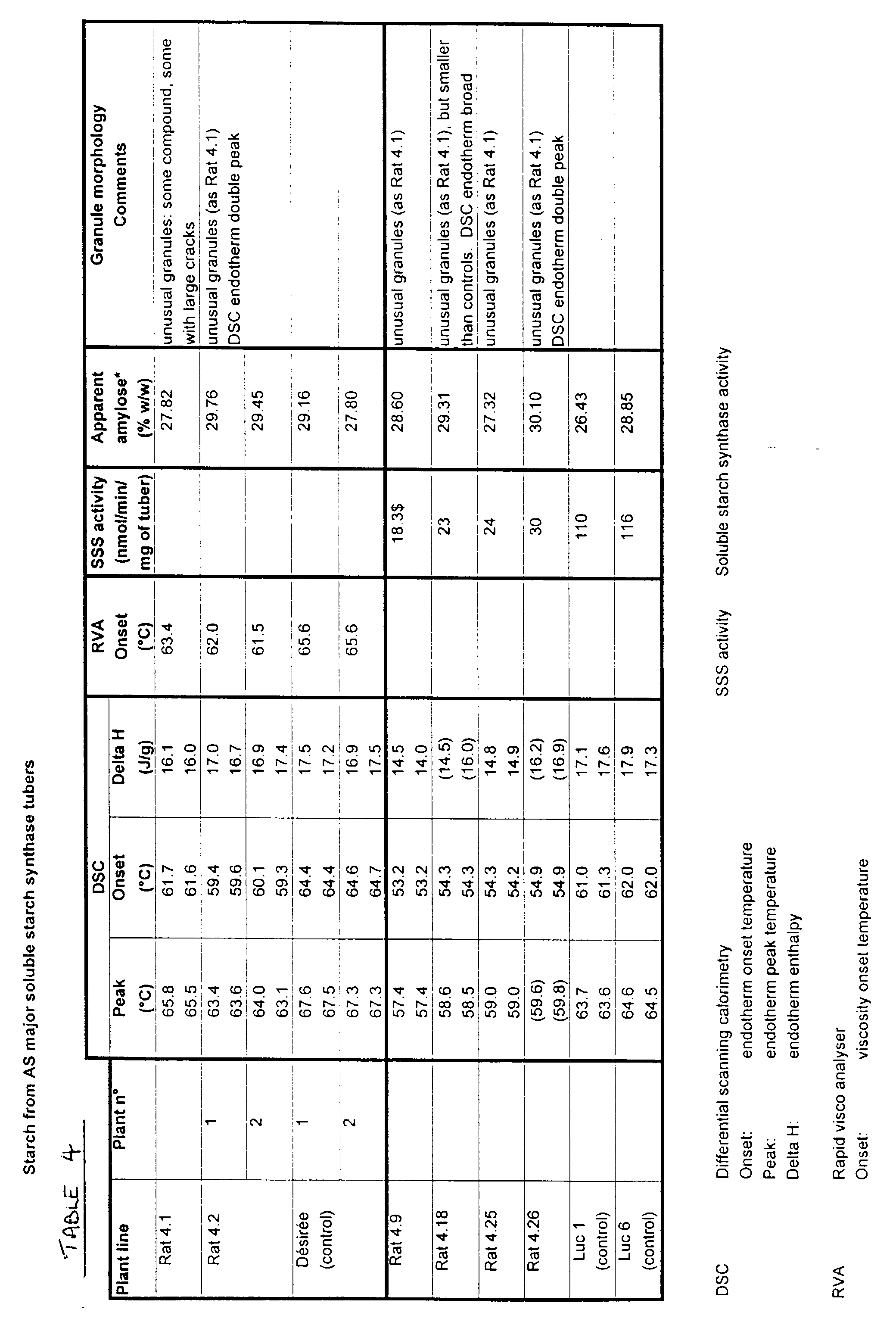

## Claims

1. Altered starch extracted from a transformed potato plant or the progeny thereof, which starch, as extracted, has a viscosity onset temperature as determined by differential scanning calorimetry which is reduced by at least 5°C compared to starch extracted from equivalent, non-transformed plants.

2. Altered starch according to claim 1, wherein the viscosity onset temperature is reduced by at least 7°C compared to starch extracted from equivalent, non-transformed plants.

3. Altered starch extracted from a transformed potato plant or the progeny thereof, which starch, as extracted, has a viscosity onset temperature as determined by differential scanning calorimetry, of less than 60°C.

4. Altered starch according to claim 3 which, as extracted, has a viscosity onset temperature of less than 55°C.

5. Altered starch according to any one of claims 1-4 which, as extracted, has a reduced endotherm peak temperature (as extracted) as determined by differential scanning calorimetry compared to starch extracted from equivalent, non-transformed plants.

6. Altered starch according to any one of claims 1-5 which, as extracted, has an endotherm peak temperature as determined by differential scanning calorimetry which is reduced by at least 5°C compared to starch extracted from equivalent, non-transformed plants.

7. Altered starch according to any one of claims 1-6 which, as extracted, has an endotherm peak temperature as determined by differential scanning calorimetry, of less than 59°C.

8. Altered starch according to any one of the preceding claims, having a substantially normal amylose content.

9. A polypeptide obtainable from a soluble extract of potato tubers and having starch synthase activity, in substantially isolated form.

10. A polypeptide according to claim 9, having an apparent molecular weight, as judged by SDS-PAGE, in the range of 100-140 kDa, or a functional equivalent thereof.

11. A polypeptide according to claim 9 or 10, having an apparent molecular weight, as judged by SDS-PAGE, of 140 kDa.

12. A polypeptide according to claim 9 or 10, having an apparent molecular weight, as judged by SDS-PAGE, of 120 kDa.

13. A polypeptide according to claim 9 or 10, having an apparent molecular weight, as judged by SDS-PAGE, of 110 kDa.

14. A polypeptide according to claim 9 or 10, obtainable from developing tubers of S. *tuberosum* cultivar Desirée, having an apparent molecular weight, as judged by SDS-PAGE, of 100 kDa.

15. A polypeptide according to any one of claims 9-14, comprising the amino acid sequence shown in Figure 6.

16. A nucleic acid sequence comprising at least 200bp and exhibiting at least 80% sequence identity with the corresponding region of the DNA sequence shown in Figure 6, operably linked in the sense or antisense orientation to a promoter operable in a plant.

17. A sequence according to claim 16 comprising at least 300-600bp.

18. A sequence according to claim 16 or 17, exhibiting at least 85% sequence identity with the corresponding region of the DNA sequence shown in Figure 6.

19. A sequence according to any one of claims 16, 17 or 18 exhibiting at least 90% sequence identity with the corresponding region of the DNA sequence shown in Figure 6.

20. A sequence according to any one of claims 16-19, comprising a 5' and/or a 3' untranslated region.

21. A sequence according to any one of claims 16-20, encoding at least a portion of a polypeptide in accordance with any one of claims 9-15.

22. A sequence according to any one of claims 16-21, excluding sequences disclosed in WO 96/15248.

23. A nucleic acid construct comprising the nucleic acid sequence of any one of claims 16-22.

24. A host cell into which has been introduced a nucleic acid sequence according to any one of claims 16-22.

25. A host cell according to claim 24, wherein the nucleic acid sequence is introduced in a construct according to claim 23.

26. A host cell according to claim 24 or 25, wherein the introduced sequence is integrated into the host cell genome.

27. A plant host cell according to any one of claims 24, 25 or 26.

28. A plant or part thereof, into which has been introduced a nucleic acid sequence according to any one of claims 16-22, or the progeny of such a plant or part thereof.

29. A plant or part thereof according to claim 28, wherein the plant is selected from the group consisting of : potato, tomato, rice, wheat, peal cassava, sweet potato, barley, oat and maize.

30. A plant according to claim 28 or 29, comprising starch in accordance with any one of claims 1-8.

31. Starch extracted from a plant according to claim 28 or 29.

32. Starch according to claim 31, having altered properties, as extracted, relative to starch extracted from equivalent but untransformed plants.

33. Starch according to claim 31 or 32, and in accordance with any one of claims 1-8.

34. A method of producing altered starch from transformed potato plants or their progeny, the method comprising extracting starch from a potato plant, at least the tubers of which comprise a nucleic acid sequence in accordance with any one of claims 16-22, said sequence having been artificially introduced into the potato plant or a predecessor thereof.
